# EUROPÄISCHE PATENTSCHRIFT

(11) **EP 1 735 068 B1**
(45) Veröffentlichungstag und Bekanntmachung des Hinweises auf die Patenterteilung: **06.03.2019**
(21) Anmeldenummer: 05735782.4
(22) Anmeldetag: 29.03.2005
(51) Int. Cl.: B01D 3/00, B01D 3/14, B01D 3/22, B01D 3/32

(54) **VERFAHREN ZUR DESTILLATION EINES POLYMERISATIONSFAEHIGEN STOFFES**
PROCESS FOR THE DESTILLATION OF A POLYMERIZABLE MATERIAL
PROCEDE POUR LA DISTILLATION D'UN MATERIAU POLYMERISABLE

(30) Priorität: 29.03.2004 DE 102004015714
(43) Veröffentlichungstag der Anmeldung: 27.12.2006
(73) Patentinhaber: Evonik Degussa GmbH, 45128 Essen (DE)
(72) Erfinder: MOSLER, Jürgen, 44577 Castrop-Rauxel (DE); SPARENBERG, Hans-Günter, 45770 Marl (DE); PAPAJEWSKI, Ludwig, 45770 Marl (DE); SELBACH, Arndt, 67246 Dirmstein (DE); LEISTNER, Jörg, 45721 Haltern am See (DE); KOHN, Jürgen, 47495 Rheinberg (DE)
(74) Vertreter: Lang, Arne
(86) Internationale Anmeldenummer: PCT/EP2005/003271
(87) Internationale Veröffentlichungsnummer: WO 2005/092464

(56) Entgegenhaltungen:
- DE-A1- 2 305 564
- DE-B- 1 192 621
- DE-C1- 19 929 407
- US-B1- 6 641 700

## Beschreibung

Die Erfindung betrifft ein Verfahren zur Herstellung eines polymerisationsfähigen Stoffs.
Als polymerisationsfähige Stoffe kommen allgemein alle der radikalischen Polymer eingesetzten Monomere in Frage. Hierunter fallen Methacrylsäure Acrylsäure, Styrol oder α-Methylstyrol, insbesondere sind Methacrylsäure oder Acrylsäure, nachfolgend gemeinsam als "(Meth)Acrylsäure" bezeichnet, zu nennen, wobei Acrylsäure besonders bevorzugt ist.
Die Anforderungen an die Reinheit von großtechnisch zur Polymerisation eingesetzten Monomeren steigen zunehmend. Auch an Massenkunststoffen werden immer höhere Reinheitsanforderungen gestellt. Dieses gilt insbesondere für im Medizin- oder Hygienebereich eingesetzte Polymere. Wasser oder wässrige Flüssigkeiten absorbierende Polymere, die allgemein als "Superabsorber" bezeichnet werden, sind ein wichtiger Bestandteil von vielen Produkten im Medizin- oder Hygienebereich. Bevorzugt werden Superabsorber in Windeln, Damenbinden und Inkontinenzartikeln eingesetzt. Superabsorber sowie andere Kunststoffe werden häufig durch radikalische Polymerisation von eine Doppelbindung aufweisenden Monomeren erhalten. Derartige eine Doppelbindung aufweisende Monomere wie (Meth)acrylsäure sind daher sehr reaktive Substanzen, die bei thermischer Belastung spontan zur radikalischen Polymerisation neigen.

Die Destillation ist eine zum Erzielen hoher Reinheiten geeignete und großtechnisch bewährte Aufarbeitungsmethode. Wegen der bei der Destillation auftretenden thermischen Belastung des zu reinigenden Monomers, neigt diese jedoch leicht zu unerwünschten Polymerisation. Hierbei bilden sich trotz Zugabe von Inhibitoren zunächst meist in Totzonen durch Überhitzung und zu lange Verweilzeiten des Monomers Polymerisationskeime, die im Laufe der Zeit wachsen und zu einer immer mehr zunehmenden Bildung von unerwünschtem Polymer führen, dass zu einer Stillegung des Destillationsbetriebes und zu einer aufwendigen Reinigung der Destillationsapparatur führt, die sehr kostenträchtig und mit einer erheblichen Belastung für Mensch und Umwelt verbunden ist.

Destillationskolonnen sind beispielsweise aus WO 00/53561 bekannt. In dieser Schrift wird zwar der Einsatz von "Dual-Flow-Böden" als Kolonnenböden offenbart, mit denen die Trennleistung erhöht werden soll, es werden jedoch keine Maßnahmen offenbart, mit denen den vorstehenden Nachteilen begegnet werden soll.

Grundsätzlich ist es Aufgabe der vorliegenden Erfindung, die mit dem Stand der Technik verbundenen Nachteile zu überwinden.

Dabei ist es insbesondere Ziel dieser Erfindung, den zeitlichen Abstand von Wartungsintervallen derartiger Destillationskolonnen zu verlängern, wobei bevorzugt auch die Stillstandszeiten deutlich reduziert werden.

Eine weitere Aufgabe liegt darin, einen möglichst hohen Reinigungsgrad im Verlaufe des Destillationsvorgangs über mehrere Trennböden zu erzielen, wobei bei jeder Trennstufe exakt reproduzierbare bzw. vorbestimmte chemische Vorgänge ablaufen.

Weiter ist es Aufgabe der Erfindung, eine homogene Verteilung des polymerisationsfähigen Stoffes in flüssiger bzw. gasförmiger Phase in einer Trennstufe bereitzustellen, ohne dass es zur Bildung von Polymer-Ablagerungen kommt.

Die oben genannten Aufgaben werden gelöst durch ein Verfahren nach Patentanspruch 1.

Der Trennboden für eine Kolonne zur Destillation eines polymerisationsfähigen Stoffes umfasst zumindest eine Bodenplatte mit einer Vielzahl von Öffnungen und mindestens einen Aufsatz, der die Vielzahl von Öffnungen in Gruppen aufteilt, wobei mit dem mindestens einen Aufsatz für ein Fluid durchströmbare Durchlässe gebildet sind.
Die Bodenplatte umfasst bevorzugt metallisches Material, allerdings sind auch andere, temperatur- und säurebeständige Materialien einsetzbar. Die äußere Gestalt der Bodenplatte ist mit Rücksicht auf die Destillationskolonne zu wählen, so dass diese beispielsweise runde, rechteckige oder ähnliche Formen aufweisen kann. Die Bodenplatte ist mit einer Vielzahl von Öffnungen versehen, womit insbesondere gemeint ist, dass ein Flüssigkeits- und/oder Gasaustausch durch die Bodenplatte hindurch möglich ist. Dabei sind die Öffnungen bevorzugt so groß, dass ein Aufstauen des flüssigen Stoffes möglich ist, sich bevorzugt sogar eine Sprudelschicht zum Wärme- und Komponentenaustausch zwischen dem flüssigen und dampfförmigen Stoffes bildet. Die Ausrichtung der Öffnungen bezüglich der Bodenplatte ist grundsätzlich frei wählbar und mit den Bedingungen im Inneren der Kolonne abzugleichen. So ist es beispielsweise möglich, eine gleichmäßige Verteilung der Öffnungen über die gesamt Bodenplatte bereitzustellen, allerdings kann es unter Umständen auch erforderlich sein, eine hiervon abweichende Gestaltung der Bodenplatte vorzunehmen. Dies wäre beispielsweise dann der Fall, wenn Randbereiche der Bodenplatte zur Lagerung in der Kolonne benötigt werden. Ähnliches gilt zum Beispiel, wenn die Bodenplatte aufgrund ihrer räumlichen Ausdehnung von einer Seite her gestützt werden muss und die Stützen derartige Öffnungen verschließen würden. Sodann ist es vorteilhaft, in diesen Bereichen der Bodenplatte keine Öffnungen vorzusehen.

Nun wird vorgeschlagen, dass der Aufsatz die Vielzahl von Öffnungen in Gruppen aufteilt, wobei mit dem mindestens einen Aufsatz für ein Fluid durchströmbare Durchlässe gebildet sind. Eine vordergründige Funktion dieses Aufsatzes ist es, die Bewegung der Flüssigkeit, die sich auf der Bodenplatte angesammelt hat, so zu beeinflussen, dass einerseits eine hohe Fließgeschwindigkeit vermieden wird, andererseits aber auch keine "Totzonen" gebildet werden, in denen relativ lange Verweilzeiten der Flüssigkeiten herrschen.

Besonders hohe Fließgeschwindigkeiten können beispielsweise auftreten, wenn der durch die Bodenplatte hindurchtretende gasförmige Stoff nicht vollkommen gleichmäßig über den Querschnitt der Bodenplatte eingeleitet wird, sondern in einem Bereich eine besonders starke Quelle gebildet ist. Dann kann es zu einer wellenförmigen Anregung der Flüssigkeit kommen. Um zu verhindern, dass sich eine derartige Wellenfront über die gesamte Bodenplatte ausbreitet und somit zu unterschiedlichen Flüssigkeitspegeln auf der Bodenplatte führt, dient der Aufsatz als eine Art Wellenbrecher, indem obere Flüssigkeits- bzw. Dampfblasenschichten schwingungsberuhigt werden.

Allerdings gewährleistet die räumlich strikt voneinander abgegrenzte Aufteilung der Bodenplatte in verschiedene Sektoren nicht zwingend ein besseres Fließverhalten der Flüssigkeit. Vielmehr werden durch die Aufteilung unter Umständen Gruppen von Öffnungen gebildet, die weniger mit dem polymerisationsfähigen Stoff beaufschlagt werden, so dass sich hinsichtlich der Sektoren unter Umständen unterschiedliche Destillationsgrade ergeben. Aus diesem Grund wird vorgeschlagen, dass die Flüssigkeit zwar durch den Aufsatz in ihrer Bewegungsfreiheit eingeschränkt, aber hinsichtlich der Erreichbarkeit einer Vielzahl von Öffnungen anderer Sektoren (insbesondere aller Öffnungen) nicht begrenzt wird. Dies wird dadurch gewährleistet, dass dennoch mit dem Absatz für ein Fluid durchströmbare Durchlässe gebildet sind. Die Durchlässe gewährleisten, dass das Fluid vorzugsweise zu jeder Öffnung der Bodenplatte hinströmen kann.

Die Gestalt des Durchlasses ist auch hier wieder unter Berücksichtigung der Flüssigkeit, des polymerisationsfähigen Stoffes, der Kolonne oder deren Betrieb zu wählen. Beispielhaft seien an dieser Stelle runde Durchlässe, rechteckige Durchlässe, Schlitze, etc. genannt. Dabei sei auch klargestellt, dass die Durchlässe vollständig oder teilweise von dem Aufsatz begrenzt bzw. gebildet sein können. Grundsätzlich ist es bei der Anordnung derartiger Durchlässe vorteilhaft, diese in unteren, d.h. bodenplattennahen, Bereichen anzuordnen. Auf diese Weise wird sichergestellt, dass einerseits die Fortpflanzung der Wellenbewegung der oberen Flüssigkeits- bzw. Fluidschichten unterbrochen wird, während tieferliegende Schichten nahe verschiedener Gruppen von Öffnungen miteinander kommunizieren können. Dabei ist darauf zu achten, dass die Durchlässe so gestaltet sind, dass möglichst wenige, bevorzugt keine, Totzonen gebildet sind, wobei hiermit Zonen gemeint sind, in denen das Fluid eine relativ lange Verweilzeit hat.

Die Flüssigkeit neigt in derartigen Totzonen zur Polymerisation, wobei dies langfristig zur zumindest teilweisen Verstopfung der Öffnungen führen kann. Das hat zur Folge, dass der Gas- bzw. Flüssigkeitsaustausch durch eine kleinere Anzahl von Öffnungen nur noch erfolgt, wobei der zunehmende Gasdruck eine zusätzliche Schwingungsanregung der Flüssigkeit zur Folge hat. Bei zunehmender Polymerisation des Stoffes kann der Destillationsschritt nicht mehr in der gewünschten Qualität durchgeführt werden, wodurch Reinigungs- bzw. Wartungsmaßnahmen erforderlich sind. Dies wiederum hat zur Folge, dass der Destillationsprozess unterbrochen und die Kolonne stillgelegt werden muss. Die Trennböden müssten in einem aufwendigen Verfahren wieder gereinigt und anschließend montiert werden. Durch den hier vorgeschlagenen Aufsatz auf der Bodenplatte können derartige, aufwendige Maßnahmen zumindest über einen längeren Zeitraum hinaus aufgeschoben werden. Aufgrund der Tatsache, dass an deutlich wenigeren Stellen Polymer-Ablagerungen auftreten, ist die Reinigung auch schneller durchführbar. Das bedeutet, dass die Destillationskolonne einerseits über einen längeren Zeitraum mit den gewünschten Destillationsergebnissen einsatzbereit ist, andererseits aber auch die Reinigungsprozedur schneller ausgeführt werden kann und somit die Stillstandszeiten der Destillationskolonne verkürzt sind.

Gemäß einer vorteilhaften Weiterbildung des Trennbodens erstrecken sich die Durchlässe von der Bodenplatte aus über eine Höhe im Bereich von 1 mm bis 100 mm, vorzugsweise von 5 mm bis 50 mm und besonders bevorzugt von 10 mm bis 30 mm. Das bedeutet insbesondere, dass die Durchlässe nur teilweise durch den Aufsatz begrenzt sind. Sie sind in diesem Falle zumindest auch teilweise von der Bodenplatte begrenzt. Dabei ist es möglich, dass die Durchlässe nur von Bodenplatte und dem Aufsatz begrenzt sind, allerdings können auch weitere Bauteile zur Begrenzung der Durchlässe herangezogen werden. Die Höhe ist dabei so gewählt, dass sich das Fluid in Bodenplattennähe relativ unbehindert bewegen bzw. fließen kann, und es somit ein gleichmäßiges. Beströmen der Öffnungen mit der Flüssigkeit bzw. dem Fluid gewährleistet ist. Gleichzeitig sind die bodenplattenfernen Flüssigkeits- bzw. Fluidschichten von dem Aufsatz durchzogen.

Gemäß einer bevorzugten Ausgestaltung umfasste der mindestens eine Aufsatz zumindest einen gerade verlaufenden Steg. Eine solche Ausgestaltung des Aufsatzes ist insbesondere dann zu wählen, wenn die Bodenplatte ebenfalls geradlinig verlaufende Kanten hat, also insbesondere rechteckig oder quadratisch ist. Dadurch ist es auf einfache Weise möglich, die Öffnungen in der Bodenplatte in Gruppen nahezu gleicher Anzahl aufzuteilen. Unter Umständen ist es jedoch auch erforderlich, dass der Aufsatz, zusätzlich zu gerade verlaufenden Stegen bzw. Abschnitten, gebogene Leitflächen aufweist, die eine solche Strömung des auftreffenden Fluids bewirken, dass Totzonen nahe dem Aufsatz vermieden werden. Die Orientierung des Steges in Bezug auf die Bodenplatte ist grundsätzlich frei wählbar. Das bedeutet auch, dass sich der gerade verlaufende Steg über die gesamte Bodenplatte oder aber auch nur Teilbereiche der Bodenplatte erstreckt.

Gerade in diesem Zusammenhang ist es vorteilhaft, dass der Aufsatz zumindest eine der folgenden Konfigurationen aufweist:
(a) es sind mehrere Aufsätze mit einem Steg vorgesehen;
(b) es ist ein Aufsatz mit mehreren, vorzugsweise miteinander verbundenen Stegen vorgesehen.

Das bedeutet insbesondere, dass der Aufsatz als Bausatz mit einer Mehrzahl verschiedener oder gleichartiger Stege aufgebaut sein kann, andererseits aber auch einstückige Aufsätze bzw. Verbundsysteme aus mehreren, miteinander vorzugsweise fügetechnisch verbundenen, Stegen zur Herstellung geeignet sind. Die Stege können bei derartigen Verbundsystemen lösbar oder durch Stoffschluss miteinander verbunden sein. Als ein Unterscheidungskriterium bei diesen beiden Konfigurationen kann beispielsweise herangezogen werden, dass die mehreren Aufsätze umfassend jeweils einem Steg lediglich über die Bodenplatte bzw. andere Komponenten der Kolonne miteinander in Kontakt sind, während bei einem einstückigen, komplexeren Aufsätzen bzw. den zu einem Aufsatz miteinander verbundenen Stegen eine direkte Verbindung bzw. Konnektierung über Bestandteile der Stege bzw. des Aufsatzes selbst gebildet ist. Während die Alternative (a) eine sehr flexible Anordnung der Stege zueinander ermöglicht, hat die Konfiguration (b) den Vorteil, dass sich diese einfach und mit geringem Zeitaufwand montieren lässt. Unter diesen Gesichtspunkten ist es unter Umständen vorteilhaft, dass in einem zentralen Bereich ein komplexer Aufsatz aus mehreren Stegen eingesetzt wird, während in den Randbereichen, je nach Form bzw. Gestalt des Aufsatzes oder der Kolonne zusätzliche Einzel-Stege zur Vervollständigung des ersten Aufsatzes positioniert werden. Somit kann sichergestellt werden, dass beispielsweise Gruppen von Öffnungen mit im wesentlichen gleicher Anzahl gebildet werden.

Gemäß einer vorteilhaften Weiterbildung hat der mindestens eine Steg eine Länge, wobei die Mehrzahl von Durchlässen zumindest teilweise durch wenigstens eines der folgenden Elemente begrenzt ist:
(a) wenigstens eine Strebe, die Teil des Steges ist;
(b) wenigstens einen Abstandshalter, der ein separates Teil ist;
wobei diese Elemente eine Breite aufweisen, und die Summe der Breiten der Elemente kleiner als 80 %, bevorzugt kleiner 60 %, insbesondere kleiner 40%, der Länge des Steges ist. Neben der Möglichkeit, den genannten Aufsatz mit einem bestimmten Abstand bezüglich der Bodenplatte an der Kolonne bzw. den Behälter zu befestigen wird hier bevorzugt eine leicht montierbare Baueinheit mit der Bodenplatte vorgeschlagen. Das bedeutet insbesondere, dass die Bodenplatte mit dem mindestens einen Aufsatz gemeinsam in die Kolonne eingebaut werden kann. Das hat zur Folge, dass der mindestens eine Aufsatz auf der Bodenplatte selbst zu befestigen ist. Hier wird nun vorgeschlagen, dies über Streben oder Abstandshalter oder beide Elemente zu realisieren.

Die Streben, die selbst Teil des Steges sind, können beispielsweise durch formgebende Verfahren hergestellt werden, wie beispielsweise Gießen, Fräsen oder dergleichen. Daraus folgt, dass die Streben aus demselben Material wie der Steg selbst gebildet sind. Unter Umständen ist es jedoch vorteilhaft, dass die Streben und der Steg selbst aus verschiedenen Materialien aufgebaut ist, wobei zum Beispiel zunächst der Steg einstückig hergestellt wird und anschließend die Stege unter Einsatz eines fügetechnischen Fertigungsverfahrens (lösbar oder unlösbar) mit dem Steg verbunden werden.

Alternativ oder in Kombination dazu können separate Abstandshalter auf der Bodenplatte positioniert werden, die selbst Mittel zur Fixierung des Steges umfassen.

Das hat den Vorteil, dass die Stege beispielsweise alle im wesentlichen rechteckig ausgebildet sein können, wobei durch jeweils unterschiedliche Ausgestaltungen der Abstandshalter die gewünschten Höhen der Durchlässe generiert werden können. Das hat eine kostengünstige Herstellung der Stege einerseits und einer einfachen verfahrensabhängige Anpassung der Durchlässe zur Folge.

Die Streben bzw. Abstandshalter dienen insbesondere zur Stabilisierung bzw. Positionierung der Strebe in Relation zur Bodenplatte. D.h., dass der Steg beispielsweise in den Randbereichen der Bodenplatte mittels jeweils einer Strebe bzw. einem Abstandshalter auf der Bodenplatte angeordnet ist, während in mittleren Bereichen je nach Länge des Steges ein zusätzliches Element (Strebe bzw. Abstandshalter) vorgesehen ist. Diese erstrecken sich dann bevorzugt bis zur Bodenplatte, unter Umständen können die Streben aber auch kürzer ausgeführt sein, um ein bestimmtes Profil der Durchlässe zu generieren, welches beispielsweise vorteilhafte Einflüsse bezüglich des Strömungsverhalten der Flüssigkeit aufweist.

Wie bereits erwähnt, ist es besonders vorteilhaft, dass die Summe der Breiten der Streben bzw. Abstandshalter kleiner als 80%, vorzugsweise kleiner als 60% und besonders bevorzugt kleiner als 40% der Länge des Steges ist. Es ist somit bevorzugt, dass möglichst wenige und bevorzugt relativ schlanke Elemente zur Stabilisierung des Steges einzusetzen sind. Das gewährleistet, dass die Strömung des Fluids in Bodenplattennähe sich relativ unbehindert fortpflanzen kann. Hierbei ist es unter Umständen vorteilhaft, die Stege bzw. Abstandshalter mit einem strömungstechnisch günstigen Profil auszustatten, insbesondere geeignete Umströmungskanten zu bilden (z.B. runde Querschnittsformen). Entsprechend den auftretenden Strömungsgeschwindigkeit kann es unter Umständen erforderlich sein, eine erhöhte Anzahl von Streben bzw. Abstandhaltern vorzusehen, diese aber relativ dünn auszuführen, während bei anderen Anwendungen unter Umständen jeweils eine Strebe bzw. ein Abstandshalter im Randbereich der Bodenplatte ausreicht, der zentrale Bereich aber frei von Streben bzw. Abstandshaltern ist. Zur Klarstellung sei hier noch festgehalten, dass die Länge des Steges die längste Ausdehnung in einer Richtung beschreibt und die Breiten der Strebe bzw. der Abstandshalter in die gleiche Richtung zu bestimmen ist, wobei die Breite und die Länge in einer Ebene oder in zueinander parallelen Ebenen angeordnet sein können.

Gemäß noch einer weiteren Ausgestaltung ist der Trennboden so ausgeführt, dass der mindestens eine Aufsatz Sektoren der Bodenplatte mit jeweils einer Gruppe von Öffnungen bildet, wobei die Sektoren bevorzugt eine Fläche im Bereich von 1,2 m² bis 0,3 m², bevorzugt 1,0 m² bis 0,5 m² und insbesondere 0,8 m² bis 0,6 m², umfassen. Grundsätzlich können derartige Trennböden mit einer einteiligen oder einer mehrteiligen Bodenplatte aus mehreren Einzelbodenplatten gebildet sein, wobei grundsätzlich sichergestellt ist, dass eine Unebenheit der Bodenplatte von kleiner 3mm/m, insbesondere kleiner 2mm/m und bevorzugt kleiner 1 mm/m realisiert ist. Eine runde Bodenplatte hat bevorzugt insgesamt eine Durchmesser im Bereich von 2 m bis 7 m, insbesondere 3 m und 5 m. Dabei haben die Öffnungen in der Bodenplatte bevorzugt einen Durchmesser von 15 mm bis 40 mm, insbesondere von 20 mm bis 30 mm. Weiter wird vorgeschlagen, dass der Trennboden eine Deckplatte hat, wobei diese bevorzugt in einer Entfernung im Bereich von 60 mm bis 200 mm, vorzugsweise von 80 mm bis 150 mm und besonders bevorzugt von 100 mm bis 120 mm von der Bodenplatte angeordnet ist und insbesondere ebenfalls eine Vielzahl von Öffnungen aufweist.

Die Deckplatte hat eine Mehrzahl unterschiedlicher Funktionen. So ist beispielsweise eine Funktion darin zu sehen, dass auf diese Weise verhindert oder zumindest deutlich reduziert wird, dass der aufsteigende dampfförmige Stoff teile des flüssigen, auf der Bodenplatte angesammelten Stoffes mitreißt. Dabei wird die abgeschiedene Flüssigkeit, die sich beim Aufsteigen des dampfförmigen Stoffes an der Deckplatte anlagert wieder gleichmäßig verteilt und der Bodenplatte darunter wieder zugeführt. Zur Verstärkung dieses Effekt weist die Deckplatte Öffnungen auf, die nicht parallel zur bevorzugten Strömungsrichtung des dampfförmigen Stoffes verlaufen, sondern schräg dazu, z.B. mit einem Winkel zwischen 10° und 50°. Dadurch wird ein intensiverer Kontakt des Stoffes mit der Deckplatte ermöglicht. Diese vorteilhaften Effekte führen einzeln und besonders in Kombination miteinander zu einer Erhöhung des Wirkungsgrades des Trennbodens. Weiterhin begünstigt die Deckplatte unter Umständen wenigstens den Stoff- oder den Wärmeaustausch, wodurch eine weitere Verbesserung der Trennleistung geschaffen wird. Gleichzeitig wird eine gleichmäßige Abströmung des dampfförmigen Stoffes weg von dem gerade durchdrungenen Trennboden hin zum nächsten ermöglicht. Somit wirkt die Deckplatte wie ein Strömungsgleichrichter, der eine gleichmäßige Anströmung des folgenden Trennbodens gewährleistet. Demnach kann die Deckplatte genau wie ein solcher Strömungsgleichrichter mit einer Gitter- oder Wabenstruktur oder einer Lochplatte ähnlich der Bodenplatte ausgeführt sein. Gerade bei der Gestaltung der Deckplatte gemäß einer Wabenstruktur aus mehreren strukturierten Blechlagen, die durchströmbare Kanäle bilden hat die Deckplatte eine Dicke von mindestens 50 mm, bevorzugt mindestens 100 mm, insbesondere 150 mm, jedoch besonders bevorzugt nicht größer als 300 mm.

Gemäß einer vorteilhaften Weiterbildung des Trennbodens umfasst dieser eine Halterung, welche wenigstens einen massiven Träger aufweist, der mit der dem mindestens einen Aufsatz abgewandten Seite der Bodenplatte in Kontakt ist, bevorzugt über mindestens eine Abmessung im Bereich von 200 mm bis 1000 mm, vorzugsweise von 350 mm bis 800 mm und besonders bevorzugt von 400 mm bis 600 mm. Dabei ist es auch möglich, dass die Träger zumindest teilweise im wesentlichen senkrecht zueinander ausgerichtet und ggf. mit Verbindungselementen miteinander verbunden sind, wobei die Träger in unterschiedliche Richtung mit voneinander verschiedenen Abmessungen versehen sein können.

Die genannte Halterung kann eine Mehrzahl von Funktionen übernehmen, wobei hier insbesondere wenigstens eine der beiden folgenden Funktionen im Vordergrund steht: die Fixierung des Trennbodens in Relation zur Kolonne bzw. weiteren Trennböden, oder die Erhöhung der Steifigkeit des Trennbodens. Dient diese Halterung zur Fixierung, so sind diese bevorzugt so angeordnet, dass sie sich bis zum Randbereich des Trennbodens hin erstreckt, so dass sie mit Komponenten benachbarter Bauteile (z.B. der Kolonne oder weiteren Trennböden) verbunden werden kann. Die Funktion betreffend die Erhöhung der Steifigkeit des Trennbodens tritt insbesondere dann zunehmend in den Vordergrund, wenn der Trennboden bzw. alle Bodenplatten gemeinsam eine Fläche von mehr als 10 m², bevorzugt mehr als 13 m² und besonders bevorzugt mehr als 15 m² überspannt. Bei zunehmender Größe des Trennbodens wird durch den Einsatz der Halterungen sichergestellt, dass die Bodenplatte dennoch eben ist und dass in zentralen Bereichen ein Absenken der Bodenplatte im Verhältnis zu Randbereichen vermieden wird. Derartige Unebenheiten der Bodenplatte hätten zur Folge, dass in Bodenplattennähe Grenzschichten gebildet werden würden, die eine reduzierte Fließneigung haben und somit zur Polymerisation neigen. Da diese Unebenheiten relativ großflächig wären, würde unter Umständen eine sehr große Anzahl von Öffnungen durch die Polymerisationsablagerungen verstopft. Diese, die Funktionsfähigkeit der Destillationskolonne einschränkenden Vorgänge können durch den Einsatz der hier vorgeschlagenen Halterungen vermieden werden.

Diesbezüglich sei darauf hingewiesen, dass unter einem massiven Träger insbesondere ein Träger ohne Hohlräume, also Räume die vollständig vom Material des Trägers umgeben sind, in dessen Inneren zu verstehen ist, wobei hiervon herstellungsbedingte Einschlüsse oder Lunker ausgenommen sind. Vorzugsweise sind die Träger auch nicht so gestaltet, dass zwar steife, aber dennoch hohlraumbildende Profile vorgesehen sind. Zwar ist es grundsätzlich möglich, derartige Halterungen aus U-Profilen mit relativ dünnen Wänden herzustellen, allerdings werden hier relativ dickwandige, massive Träger bevorzugt vorgeschlagen. Die Träger sind auf der Seite der Bodenplatte angebracht, die dem Flüssigkeitsspiegel abgewandt ist. Sie dienen demnach nicht zur direkten Strömungsbeeinflussung sondern nur gegebenenfalls als Abströmkanten bzw. -flächen für Fluid bzw. Flüssigkeit, welches durch die Öffnungen der Bodenplatte hindurchgetreten ist oder welches an der Halterung bzw. dem Trennboden kondensiert ist.

Gemäß einer weiteren Ausgestaltung hat der wenigstens eine Träger nahe der Bodenplatte mindestens eine Aussparung mit einer Ausdehnung, wobei die Summe der Ausdehnungen mindestens im Bereich von 80 % bis 30 %, vorzugsweise von 70 % bis 40 % und besonders bevorzugt von 60 % bis 55 % der Abmessung 46 liegt. Die Aussparungen haben unter anderem die Funktion, ein Durchströmen mit dem dampfförmigen Stoff zu ermöglichen und ggf. Abrisskanten für an dem Träger herabrieselnde Flüssigkeitsströmungen darzustellen. Je größer die Oberfläche eines solchen Trägers ist, desto mehr der durch die Öffnungen hindurchgetretenen Flüssigkeit kann sich dort anlagern. Somit würde auch hier die Gefahr bestehen, dass Grenzschichten gebildet werden, in denen es rasch zur Polymerisation kommt. Aus diesem Grund wird hier vorgeschlagen, dass bereits nach kurzen Strömungswegen Kanten gebildet werden, die ein weiteres Strömen entlang des Trägers infolge der Gravitation verhindern. Diese Kanten haben zur Folge, dass sich beispielsweise Tropfen bilden, die sich von dem Träger lösen und in Richtung des Unterbodens der Destillationskolonne oder dazwischenliegenden Trennböden frei fortbewegen. Um sicherzustellen, dass einerseits ein solch vorteilhaftes Abtropfen der Flüssigkeit gewährleistet ist, andererseits aber auch die steifigkeitserhöhende Eigenschaft des Trägers gewährleistet ist, wird vorgeschlagen, dass die Summe der Abmessungen in dem oben genannten prozentualen Bereich der Abmessung liegt. Auch in diesem Zusammenhang sei darauf hingewiesen, dass die Abmessung in Richtung der längsten Erstreckung des Trägers zu bestimmen ist, wobei die Ausdehnung der Aussparungen in die gleiche Richtung zu erfassen ist, so dass Ausdehnung und Abmessung in derselben bzw. in zueinander parallelen Ebenen des Trägers liegen.

Gemäß einer weiteren Ausgestaltung ist der Träger T-förmig gestaltet, wobei die mindestens eine Aussparung in dem im wesentlichen senkrecht zur Bodenplatte liegenden Trägerteil angeordnet ist. Die T-förmige Ausgestaltung bietet die Vorteile, dass mit dem oberen, im wesentlichen parallel zur Bodenplatte liegenden, Trägerteil eine Kontaktfläche hin zur Bodenplatte bereitgestellt wird, die eine ebene und relativ großflächige Auflage gewährleistet. Dabei sind ausreichend Möglichkeiten zur Verbindung mit der Bodenplatte gegeben. Diesbezüglich ist es weiter vorteilhaft, dass die Bodenplatte im Bereich der Anlage mit dem Träger keine Öffnungen aufweist. Weiterhin hat dieser im wesentlichen parallel zur Bodenplatte liegende Trägerteil den Vorteil, dass beim Hinabströmen der Flüssigkeit bereits nach einem kurzen Weg die erste Abrisskante gebildet ist, nämlich genau dort, wo der obere Trägerteil aufhört. Dabei ist der im wesentlichen senkrecht zur Bodenplatte angeordnete Trägerteil soweit von dieser Kante entfernt positioniert, dass eine Ansammlung von Flüssigkeit in Folge der Oberflächenspannung im Bereich des Aufeinandertreffens des oberen und unteren Trägerteils vermieden wird.

Allerdings ist zu berücksichtigen, dass unter Umständen der gasförmige, polymerisationsfähige Stoff an der Unterseite des im wesentlichen parallel zur Bodenplatte liegenden Trägerteils kondensiert und nun doch die Möglichkeit besteht, dass diese Flüssigkeit am im wesentlichen senkrecht zur Bodenplatte liegenden Trägerteil hinabströmt. Aus diesem Grund wird hier die Anordnung von mindestens einer Aussparung vorgeschlagen. Weiterhin ist die Vorsehung solcher Aussparungen deshalb vorteilhaft, weil auf diese Weise Material gespart und somit die Herstellungskosten, das Bauteilgewicht und auch die Mannstärke zur Montage derartiger Trennböden deutlich reduziert werden können. Unter diesen Gesichtspunkten sind die Aussparungen unter Umständen auch nach den Kriterien der Leichtbauweise, wie sie bereits vielfach eingesetzt wird, zu gestalten. Dabei können die Gesichtspunkte der Leichtbauweise durch eine entsprechende Materialwahl weiter verstärkt werden.

Gemäß einer vorteilhaften Weiterbildung des Trennbodens wird vorgeschlagen, dass der wenigstens eine T-förmige Träger einen parallel zur Bodenplatte angeordneten Fuß mit einer Dimension im Bereich von 50 mm bis 100 mm, vorzugsweise von 60 mm bis 90 mm und besonders bevorzugt von 70 mm bis 80 mm hat, wobei die mindestens eine Aussparung in einem Abstand kleiner 10 mm, insbesondere direkt am Fuß anschließend, angeordnet ist und bevorzugt eine Weite im Bereich von 40 mm bis 120 mm, vorzugsweise von 60 mm bis 100 mm und besonders bevorzugt von 75 mm bis 85 mm hat. Zur Herstellung eines solchen Trägers ist es auch möglich, mehrere Einzelteile lösbar (z.B. mit Verbindungselementen) oder unlösbar (z.B. durch Schweißverbindungen) miteinander zu verbinden. Die Dimensionierung des T-förmigen Trägers erfolgt im wesentlichen unter Berücksichtigung zweier Interessen. Einerseits ist darauf zu achten, dass der Träger in ausreichendem Maße die gewünschte Steifigkeit des Trennbodens bereitstellt, andererseits ist zu berücksichtigen, dass unter Umständen die Wärmekapazität eines solchen Trägers eine wichtige Rolle hinsichtlich des Betriebes der Destillationskolonne spielt. Das bedeutet, dass bei Temperaturschwankungen im Inneren der Kolonne nicht übermäßig große Temperaturdifferenzen zwischen dem vorbeiströmenden Gas und dem Träger auftreten dürfen, da dies eine erhöhte Gefahr der Kondensation bzw. Polymerisation des Stoffes zur Folge hat.

Weiter wird vorgeschlagen, dass der mindestens eine Träger und der mindestens eine Aufsatz senkrecht zueinander angeordnet sind. Das bedeutet, dass die Träger und der mindestens eine Aufsatz eine Art Fachwerk bilden, die eine Stabilisierung der Bodenplatte in einer Ebene zur Folge hat. Dies gilt in zunehmendem Maße wie der mindestens eine Aufsatz direkte Verbindungsbereiche mit der Bodenplatte (auch im zentralen Bereich) aufweist. Eine solche Versteifung der Bodenplatte mittels eines solchen Fachwerksystems hat zur Folge, dass die oben aufgeführten Bereiche betreffend die Dimension bzw. die Weite des T-förmigen Trägers schlanker ausgeführt sein können, bevorzugt also in Bereiche der unteren Hälfte bzw. des unteren Drittels des angegebenen Wertebereichs.

Weiter wird noch vorgeschlagen, dass der Trennboden zumindest teilweise mit einer Beschichtung versehen ist, die eine gegenüber Stahl verbesserte Gleiteigenschaft für Flüssigkeiten aufweist. Dabei sei darauf hingewiesen, dass sich eine solche Beschichtung zumindest teilweise über eine der folgenden Komponenten erstreckt: die Bodenplatte, die Halterung, der Aufsatz, die Abstandshalter. Grundsätzlich kann sich die Beschichtung ausgehend von der Oberfläche der Komponenten über wenige Mikrometern bis hin zu einigen Millimetern (bevorzugt 50 µm bis 1000 µm, insbesondere 100 µm bis 800 µm, vorzugsweise 200 µm bis 400 µm) erstrecken. Als Beschichtung werden insbesondere ein Polyfluorkohlenwasserstoff, vorzugsweise Teflon, Polyanilin-Lacke bzw. Beschichtung mit einer Metall-Ionen-freien Oberfläche (z.B. Glas) oder auch Gemische von mindestens zwei dieser Beschichtungsarten vorgeschlagen. Wird Glas als Beschichtungsmittel eingesetzt, so ist inbesondere technisches Glas, welches aus abgekühlten Schmelzen von Siliziumdioxid (SiO₂), Calciumoxid (CaO), Natriumoxid (Na₂O), gegebenenfalls mit größeren Mengen an Bortrioxid (B₂O₃), Aluminiumoxid (AL₂O₃), Bleioxid (PbO), Magnesiumoxid (MgO), Bariumoxid (BaO) oder Kaliumoxid (K₂O) erhalten wurde, bevorzugt. Vorzugswiese besteht dieses technische Glas zu mindestens 50 Gew.-%, darüber hinaus bevorzugt zu mindestens 65 Gew.-% und am meisten bevorzugt zu mindestens 80 Gew.-% aus SiO₂.

Grundsätzlich ist es auch möglich, den Trennboden zumindest teilweise aus einem nichtmetallischen Material herzustellen, so dass es einer separaten Beschichtung der oben genannten Art nicht bedarf. Neben den vorstehend genannten Materialien kann auch Kunststoff oder ein Verbundstoff (z.B. aus Teflon und Kunststoff) zur Herstellung von mindestens einer Komponente bzw. des gesamten Trennbodens herangezogen werden. Besonders vorteilhaft ist die Bereitstellung der Bodenplatte aus Teflon oder Kunststoff.

Ergänzend ist darauf hinzuweisen, es von Vorteil ist, dass der Behälter eine Mehrzahl von Trennböden aufweist und mindestens eine Sprühanlage vorgesehen ist, mit der eine Unterseite wenigstens eines Trennbodens mit dem polymerisationsfähigen Stoff besprühbar ist. Eine solche Sprühanlage entfernt wirkungsvoll an der Unterseite des Trennbodens anhaftende Teilmengen des polymerisationsfähigen Stoffes, so dass eine Polymerisation an diesen Stellen vermieden werden kann. Bevorzugt ist dabei die Ausgestaltung, bei der der unterste Trennboden der Kolonne in Reichweite einer solchen Sprühanlage positioniert ist. Dabei ist vorteilhaft, dass die Sprühanlage mit flüssigem polymerisationsfähigem Stoff aus dem Sammelbecken des Behälters versorgt wird und dieses gleichmäßig verteilt auf die Unterseite des Trennbodens gesprüht wird. Die Sprühanlage weist bevorzugt eine Mehrzahl von Düsen auf, die über den Querschnitt des Trennbodens gleichmäßig verteilte Sprühbereiche bilden. Ganz besonders bevorzugt wird die Sprühanlage mit hohem Druck betrieben, z.B. im Bereich von 2 bis 5 bar, bevorzugt um 3 bar. Weiter ist bevorzugt, dass die Sprühanlage in einer geringen Entfernung zur Unterseite des Trennbodens positioniert ist, beispielsweise höchstens 1 Meter oder 50 cm, wobei dies unter Berücksichtigung des Sprühbereichs und/oder der Anzahl der Düsen variiert werden kann. Die vorstehend beschriebene Sprühanlage ist bevorzugt in Kombination mit einem Trennboden der hier erfindungsgemäß beschriebenen Art einzusetzen. Weitere Details gehen aus der Figurenbeschreibung hervor.

Die Erfindung betrifft ein Verfahren zur Aufreinigung eines polymerisationsfähigen Stoffes, wobei der polymerisationsfähige Stoff in einer Kolonne durch den Zulauf als eine Stoff-Flüssigkeit eingebracht und in dem Innenbereich in eine zumindest teilweise Stoff-Gasphase überführt wird. Hierbei ist es bevorzugt, dass die Stoff-Gasphase einen oberhalb des Zulaufs angeordneten ersten Trennboden mit einer isotropen Dichte anströmt, wobei innerhalb einer Ebene zwischen dem Zulauf und dem ersten Trennbodens die maximale Abweichung um einen Mittelwert der isotropen Dichte höchstens 15% beträgt. Besonders vorteilhaft ist, wenn die maximale Abweichung höchstens 10% beträgt, insbesondere nur 5%. Die Ebene liegt dabei bevorzugt relativ dicht und parallel zu dem ersten Trennboden, um einen relativ kompakten Aufbau der Kolonne sicherzustellen. Die Ebene liegt dabei vorzugsweise höchstens 100 mm unterhalb des ersten Trennbodens, der Abstand der Ebene vom ersten Trennboden kann jedoch auch maximal 50 mm oder sogar nur höchstens 10 mm betragen.
Einen besonders gleichmäßige Destillation mit einer sehr geringen Polymerisationsneigung in der Kolonne ist gegeben, wenn der mindestens eine Trennboden so gestaltet ist, dass sich die isotrope Dichte auch in der flüssigen Phase des polymerisationsfähigen Stoffes einstellt. Dies lässt sich in besonderem Maße durch den Einsatz eines so genannten "Dual-Flow-Bodens" erreichen. Dabei lassen sich ggf. Strömungen der Stoff-Gasphase über die Trennböden hinweg mit der vorgeschlagenen isotropen Dichte verwirklichen, wobei die auftretende maximale Abweichung weiter deutlich reduziert wird.

Im Allgemeinen kommen alle dem Fachmann bekannten zur Polymerisation neigenden chemischen Verbindungen als polymerisationsfähige Stoffe in betracht. Bevorzugte polymerisationsfähige Stoffe sind bei der Herstellung von Massenkunststoffen verwendete Monomere wie Styrol, α-Methylstyrol, Methlmethacrylat, Butylacrylat und der gleichen. Weiterhin ist es bevorzugt, dass der in dem erfindungsgemäßen Verfahren nach eingesetzte polymerisationsfähige Stoff (Meth)Acrylsäure ist. Der Begriff "(Meth)Acrylsäure" steht vorliegend sowohl für die Verbindung mit dem Nomenklaturnamen "Acrylsäure" als auch für die Verbindung mit dem Nomenklaturnamen "Methacrylsäure", wobei Acrylsäure von beiden bevorzugt ist. Zudem ist bevorzugt, dass bei dem erfindungsgemäßen Verfahren im Innenbereich ein Absolutdruck herrscht. Dieser liegt bevorzugt im Bereich von 50 bis 400 hPa (Hektopascal), vorzugsweise 100 bis 300 hPa und besonders bevorzugt 150 bis 250 hPa im Innenbereich der Kolonne ausgesetzt wird (wobei 1 hPa = 1 mbar = 10² Newton/Meter² [N/m²] = 10² Pa).
Zudem ist es in dem erfindungsgemäßen Verfahren bevorzugt, dass die Stoff-Flüssigkeit überhitzt ist. In diesem Zusammenhang ist es bevorzugt, dass die Temperatur der Hauptkomponente der Stoff-Flüssigkeit, meist der polymerisationsfähige Stoff mindestens um 1 °C, vorzugsweise mindestens um 5 °C und besonders bevorzugt mindestens um 10 °C über der Siedetemperatur der reinen Hauptkomponente der Stoff-Flüssigkeit liegt.
Ferner betrifft die Erfindung ein Verfahren zur Herstellung eines polymerisationsfähigen Stoffs, wobei der polymerisationsfähige Stoff aus mindestens einem Edukt in einem Reaktor synthetisiert und nachfolgend einem erfindungsgemäßen Verfahren zum Aufreinigen unterzogen wird. Die Synthese des polymerisationsfähigen Stoffs ist nicht auf ein bestimmtes Verfahren beschränkt. Vielmehr kommen alle dem Fachmann bekannten Verfahren in Betracht. Bei der Synthese von Acrylsäure ist eine vorzugsweise mindestens zweistufige Gasphasenoxidationsreaktion, bei der bevorzugt in einem ersten Schritt durch katalytische Oxidation von Propylen Acrolein und in einem weiteren Schritt Acrylsäure als Gasphase erhalten wird. Diese Gasphase wir dann in einer Quencheinheit mit einer Flüssigkeit, vorzugsweise Wasser oder einer höher als Wasser siedenden organischen Verbindung oder einer Mischung daraus in Kontakt gebracht und mittelbar oder unmittelbar dem erfindungsgemäßen Verfahren zur Aufreinigung unterzogen. Einzelheiten zur Herstellung und zu weiteren Aufreingungsverfahren von Acrylsäure sind WO 02/055469 und den darin zitierten weiteren Nachweisen zu entnehmen, auf die hiermit als ein Teil dieser Offenbarung bezug genommen wird.

Die Erfindung wird nun anhand der Figuren näher erläutert, wobei die dargestellten Ausführungsbeispiele besonders bevorzugte Ausgestaltungen der Erfindung zeigen bzw. die Einordnung der Erfindung in das bekannte Umfeld der Destillations-Kolonnen veranschaulichen. Dabei sei darauf hingewiesen, dass die Erfindung nicht auf die dargestellten Ausführungsbeispiele begrenzt ist. Zudem werden unabhängig davon auch weitere Besonderheiten betreffend das technische Gebiet der Destillations-Kolonnen beschrieben.

Es zeigen:
Fig. 1 schematisch und perspektivisch den Aufbau einer Kolonne mit einem Zulauf für einen pölymerisationsfähigen Stoff;
Fig. 2 schematisch eine Schnittansicht durch eine Ausgestaltung eines Trennbodens;
Fig.3 eine schematische Ansicht auf eine weitere Ausführungsform eines Trennbodens;
Fig. 4 eine weitere schematische Ansicht einer weiteren Ausführungsform des Trennbodens im Schnitt;
Fig. 5 vereinfachte, schematische Darstellungen unterschiedlicher Ausführungsformen eines Strömungsgleichrichters;
Fig. 6 eine schematische Detailansicht einer Ausführungsform einer beschichteten Bodenplatte eines Trennbodens;
Fig.7 eine schematische Darstellung einer Anlage zur Herstellung von Acrylsäure;
Fig.8 schematisch den Aufbau einer Versuchsanordnung zur Bestimmung der Dichteverteilung;
Fig. 9 schematisch und perspektivisch eine gewellte Bodenplatte eines Trennbodens;
Fig. 10 schematisch und perspektivisch eine weitere Ausführungsform des Zulaufs mit einem Strömungsmischer;
Fig. 11 einen Teilausschnitt eines Behälters mit einer Sprühanlage im Querschnitt; und
Fig. 12 eine Draufsicht auf die in Fig. 11 gezeigte Sprühanlage.

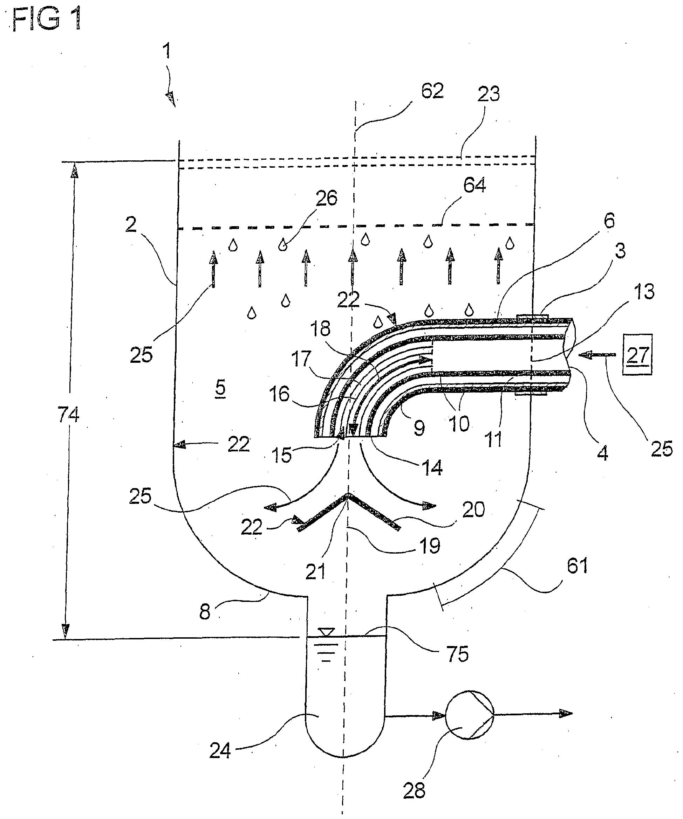

Fig. 1 zeigt schematisch und in einer Schnittansicht eine Kolonne 1 zur Destillation eines polymerisationsfähigen Stoffes, wobei diese einen Behälter 2 mit einem Unterboden 8 sowie einen Zulauf 4 für den polymerisationsfähigen Stoff umfasst. Der Zulauf 4 führt in einen Innenbereich 5 des Behälters 2. Wie im folgenden noch näher ausgeführt wird, weist die Kolonne 1 verschiedene Mittel zur gleichmäßigen Verteilung des Stoffes in dem Behälter 2 auf.

Um grundsätzlich den Strömungsverlauf des polymerisationsfähigen Stoffes nachvollziehen zu können, wird zunächst dessen Weg durch die Kolonne 1 beschrieben. Üblicherweise liegt der polymerisationsfähige Stoff zunächst als Flüssigkeit vor und wird mittels eines Erhitzers 27 in einen dampfförmigen bzw. gasförmigen Zustand transformiert bzw. überhitzt. Ausgehend von dem Erhitzer 27 strömt der Stoff in Strömungsrichtung 25 durch einen Zulauf 4 in den Innenbereich 5 des Behälters 2. Beim Eintritt oder kurze Zeit nach dem Eintritt in den Innenbereich 5 strömt der teils flüssige, teils dampfförmige Stoff weiter in Strömungsrichtung 25 (hier senkrecht nach oben anhand der Pfeile dargestellt) hin zu einem Trennboden 23, in dem eine erste Destillationsstufe verwirklicht ist. Kondensierte Bestandteile des Stoffes in Form von Tropfen 26 fallen in der Strömungsrichtung 25 entgegengesetzter Richtung wieder auf den Zulauf 4 bzw. den Unterboden 8 des Behälters 2. An der tiefsten Stelle weist der Behälter 2 ein Sammelbecken 24 auf, in dem sich das Kondensat sammelt. Dieses Sammelbecken ist mit einer Pumpe 28 verbunden, die den Abtransport des Kondensats im Sammelbecken 24 aus der Kolonne 1 erledigt.

Bei näherer Betrachtung des Zulaufs 4 ist zunächst zu erkennen, dass dieser eine Eintrittsöffnung 13 und eine Austrittsöffnung 14 hat, wobei hier die Austrittsöffnung 14 näher und im wesentlichen parallel zum Unterboden 8 des Behälters2 angeordnet ist. Der Zulauf 4 ist als separates Bauteil dargestellt, welches durch einen Anschluss 3 durch den Behälter 2 in den Innenbereich 5 hineinragt. Der Zulauf 4 weist gerade und gekrümmt verlaufende Teilbereiche auf, wobei diese hier so gestaltet sind, dass die Austrittsöffnung 14 mit ihrer Mittelachse 19 zentrisch zur Zentrumsachse 62 des Behälters 2 ausgerichtet ist. Im Inneren des Zulaufs 4 ist über eine Strecke 18 bis hin zur Austrittsöffnung 14 ein Strömungsbeeinflusser 15 angeordnet. Der Strömungsbeeinflusser 15 umfasst eine Mehrzahl von Leitblechen 16, die Kanäle 17 zur Vergleichmäßigung der Strömung des polymerisationsfähigen Stoffs im Inneren des Zulaufs 4 gewährleisten. Zentrisch zur Mittelachse 19 bzw. der Zentrumsachse 62 ist ein kegelförmig gestalteter Strömungsverteiler 20 so angeordnet, dass seine Spitze 21 der Austrittsöffnung 14 am nächsten ist. Wie in der Fig. 1 anhand der Pfeile dargestellt ist (Strömungsrichtung 25), hat die Anordnung des Strömungsverteilers 20 im Innenbereich 5 des Behälters 2 eine Umlenkung des einströmenden Stoffes zur Folge, wobei zur Unterstützung zusätzlich der Behälter 8 so gestaltet ist, dass die Leitflächen 61 die gleichmäßige Verteilung des Stoffes in dem Behälter 2 unterstützen. Weiterhin wird mit der dargestellten Anordnung des Strömungsverteilers 20 der Vorteil erzielt, dass der einströmende Stoff nicht direkt mit dem im Sammelbecken 24 gespeicherten Kondensat vermischt wird, so dass der Strömungsverteiler 20 hier sogar noch eine Schutzfunktion hat.

Bezüglich des Zulaufs 4 ist noch festzuhalten, dass dieser mit einer Mehrzahl von Mitteln zur thermischen Isolierung gegenüber dem Innenbereich 5 versehen ist, wobei diese in einen Teilbereich 9 angeordnet sind, der sich die gesamte Außenfläche 6 des Zulaufs 4 erstreckt, welche mit dem Innenbereich 5 des Behälters 2 in Kontakt ist. Der Zulauf 4 ist als doppelwandiges Rohr ausgebildet, so dass er zwei Umhüllungen 10 aufweist, die koaxial zueinander angeordnet sind. Zwischen den beiden Umhüllungen 10 ist eine thermisch isolierte Schicht 11 als Vakuum ausgeführt, wobei die Innenfläche 12 der Umhüllung 10 verspiegelt sind.

Um zu verhindern, dass sich insbesondere flüssige Bestandteile des polymerisationsfähigen Stoffes an den den Innenraum 5 begrenzenden Flächen anlagern bzw. haften bleiben, ist die gesamte Außenfläche 6 des Zulaufs 4, die gesamte Mantelfläche bzw. Spitze 21 des Strömungsverteilers 20 und auch die Innenwandung des Behälters 2 mit einer Beschichtung 22 versehen, die eine gegenüber Stahl verbesserte Gleiteigenschaft für Flüssigkeiten aufweist.

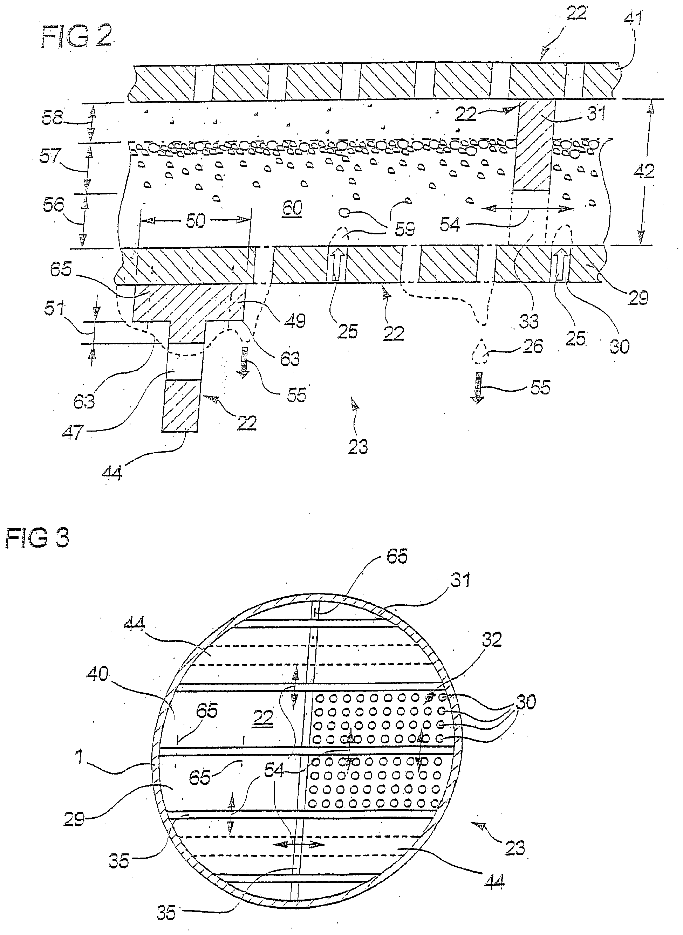

Die Fig. 2 zeigt schematisch und in einem Teilschnitt einen Trennboden 23, der eine Deckplatte 41, einen Aufsatz 31, eine Bodenplatte 29 und einen Träger 44 umfasst. Zwischen der Deckplatte 41 und der Bodenplatte 29 ist die Flüssigkeit 60 angeordnet. Durch die Öffnungen 30 der Bodenplatte 29 tritt der gasförmige, polymerisationsfähige Stoff in Strömungsrichtung 25 mit der Flüssigkeit 60 in Kontakt; wobei sich unterschiedliche Grenzschichten zwischen der Bodenplatte 29 und der Deckplatte 41 ausbilden. So lässt sich eine Flüssigkeitsschicht 56 erkennen, die im wesentlichen frei von Blasen 59 ist. Darüber ist eine Dampfblasenschicht 57 bzw. eine Art Schaumschicht angeordnet. Diese stellt praktische eine Art Grenzschicht zwischen der Flüssigkeit 60 und dem gasförmigen Volumen dar. Zwischen der Deckplatte 41 und dieser Dampfblasenschicht 57 ist weiterhin eine Tropfenschicht 58 angeordnet, wobei sich diese im wesentlichen durch einen gasförmigen Zustand des zu destillierenden Stoffes auszeichnet, der von flüssigen Tropfen 26 ausgehend von der Deckplatte 41 durchzogen ist. Während sich der gasförmige Stoff in Strömungsrichtung 25 (wie im Bild dargestellt) von unten nach oben fortbewegt, folgt die Flüssigkeit 60 der Gravitation 55 und fällt in entgegengesetzter Richtung (Gegenstrom-Prinzip) hin zum Unterboden 8 (nicht dargestellt).

Ergänzend sei an dieser Stelle darauf hinzuweisen, dass die Deckplatte 41 nicht zwingend einteilig sondern auch mehrteilig aufgebaut sein kann. Bevorzugt umfasst die Deckplatte 41 eine Vielzahl von strukturierten Blechen und/oder Kunststoff-Elementen, die zu Packungen gestapelt sind und zwischen sich (bevorzugt nicht geradlinige) Strömungspassagen bilden. Die Bleche bzw. Kunststoff-Elemente sind bevorzugt im Wesentlichen parallel zur Gravitationsrichtung angeordnet, insbesondere in einer Entfernung 42 von der Bodenplatte 29 im Bereich von 100 bis 200 mm. Die Bleche bzw. Kunststoff-Elemente sind vorteilhafterweise so bereitgestellt, dass sich eine Dicke der Deckplatte 41 bzw. der Packung von ca. 100 bis 200 mm ergibt.

Die Bodenplatte 29 weist eine Vielzahl von Öffnungen 30 auf, die mit Hilfe des Aufsatzes 31 in mehrere Gruppen 32 unterteilt werden (siehe Fig. 3). Der Aufsatz 31 ist dennoch so gestaltet, dass für das Fluid bzw. die Flüssigkeit 60 durchströmbare Durchlässe 33 gebildet sind, die in Richtung des Pfeils 54 (also im wesentlichen parallel zur Bodenplatte 29 bzw. im wesentlichen senkrecht zur Strömungsrichtung 25 des Stoffes) einen Flüssigkeitsaustausch von benachbart zueinander angeordneten Öffnungen 30 gewährleistet. Der Aufsatz 31 ist hier mit einer Beschichtung 22 versehen, die gegenüber Stahl eine verbesserte Gleiteigenschaft für Flüssigkeiten aufweist. Gleichzeitig wirkt der Aufsatz 31 als Abstandsbegrenzer bzw. Stützwand bezüglich der beiden Platten 29 und 41. Auf diese Weise ist sichergestellt, dass die Deckplatte 41 in einer vorgegebenen Entfernung 42 von der Bodenplatte 29, vorzugsweise parallel zur Bodenplatte 29, angeordnet ist. Unter Berücksichtigung der Größe bzw. Höhe 34 der Durchlässe 33 ist zu erkennen, dass diese im wesentlichen etwas kleiner als die Flüssigkeitsschicht 56 ausgebildet ist, so dass in oberhalb liegenden, nahe der Dampfblasenschicht 57 angeordneten Bereichen der Flüssigkeitsschicht 56 eine Behinderung der Strömung stattfindet, während in Nähe der Bodenplatte 29 in Richtung des Pfeils 54 relativ unbehinderte Flüssigkeitsbewegungen ermöglicht sind. Die Deckplatte 41 kann auch als Strömungsgleichrichter 64 ausgebildet sein, insbesondere als Wabenstruktur 68 mit einer Vielzahl von für ein Fluid durchströmbaren Kanälen.
Auf der dem Absatz 31 abgewandten Seite 45 ist als Halterung ein Träger 44 vorgesehen. Der Träger 44 ist T-förmig ausgebildet und weist einen im wesentlichen parallel zur Bodenplatte 29 angeordneten Fuß 49 mit einer vorgebbaren Dimension 50 sowie ein hierzu im wesentlichen senkrechten, unteren Trägerteil auf. In dem im wesentlichen senkrecht zur Bodenplatte 29 ausgerichteten Trägerteil des T-förmigen Trägers 44 sind Aussparungen 47 vorgesehen. In dem dargestellten Ausführungsbeispiel sind die Aussparungen 47 in einem Abstand 51 kleiner 3 mm angeordnet. Dadurch werden eine Vielzahl von Abrisskanten 63 gebildet, die zur Folge haben, dass herablaufendes Fluid (als Strich-Punkt-Linie dargestellt) Tropfen 26 bildet und in Richtung der Gravitation 55 sich von der Oberfläche löst. Zur Unterstützung dieses Effektes sind sowohl die Bodenplatte 29 als auch der Träger 44 mit einer Beschichtung 22 versehen, die insbesondere Teflon umfasst.
Fig. 3 zeigt schematisch und in einer Draufsicht eine weitere Ausführungsform eines Trennbodens 23. Wie sich hier erkennen lässt, überspannt der dargestellte Trennboden 23 den gesamten Innenbereich 5 der Kolonne 1 bzw. des Behälters 2. Es ist jedoch auch möglich, dass eine Mehrzahl derartiger, dann vorzugsweise rechteckiger, Trennböden 23 zu einer einheitlichen Plattform zusammengesetzt sind, die letztendlich den gesamten Innenbereich 5 der Kolonne 1 überspannt. Die hier gezeigte runde Ausführungsform des Trennbodens 23 weist eine Vielzahl von Öffnungen 30 auf, wobei diese durch den Aufsatz 31 in mehrere Gruppen 32 unterteilt werden. Der Aufsatz 31 umfasst eine Mehrzahl von Stegen 35, die in regelmäßiger Anordnung miteinander fügetechnisch verbunden sind. Der so gestaltete Aufsatz bildet Sektoren 40 der Bodenplatte 29 mit jeweils einer Gruppe 32 von Öffnungen 30. Dabei ist der Aufsatz 31 so gestaltet, dass dennoch in Richtung der Pfeile 54 ein Austausch von Flüssigkeit bzw. Fluid aus benachbarten Sektoren 40 gewährleistet ist. In gestrichelter Darstellung sind zudem die Träger 44 auf der unteren Seite 45 der mit einer Beschichtung 22 versehenen Bodenplatte 29 dargestellt. Diese sind hier direkt mit der Kolonne 1 verbunden und dienen unter anderem der Stabilitätserhöhung der Bodenplatte 29.

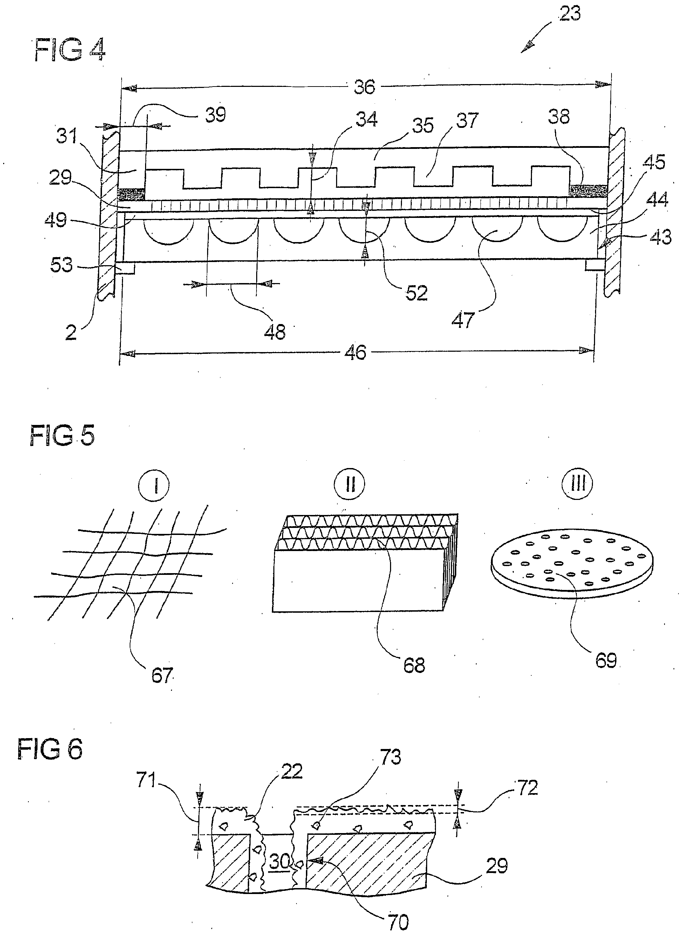

Die Fig. 4 zeigt eine weitere Schnittansicht zur Erläuterung einer Ausführungsvariante des erfindungsgemäßen Trennbodens 23. Wie sich aus Fig. 4 erkennten lässt, ist die Halterung 43 des Trennbodens 23 mit einem Träger 44 realisiert, der über Vorsprünge 53 mit dem Behälter 2 der Kolonne 1 verbunden ist, so dass eine im wesentlichen waagerechte Positionierung des Trennbodens 23 im Innenbereich 5 der Kolonne 1 sichergestellt ist. Die Vorsprünge 53 sind hier vereinfacht dargestellt. Tatsächlich können eine Mehrzahl von Justiermöglichkeiten vorgesehen sein, die eine exakte, waagerechte Ausrichtung des Trennbodens 23 im Innenraum 5 ermöglichen. Der dargestellte Träger 44 hat eine Abmessung 46 und ist T-förmig ausgeführt. Neben dem im wesentlichen senkrecht zur Bodenplatte 29 ausgerichteten Trägerteil, weist der Träger 44 einen Fuß 49 auf, der zur Auflage der Bodenplatte 29 dient. Direkt an diesem Fuß 49 schließt sich in dem senkrechten Trägerteil eine Mehrzahl von Aussparungen 47 an, wobei diese hier halbkreisartig ausgebildet sind. Die halbkreisartige Ausführung 47 ist nicht zwingend, hat aber aufgrund ihrer abgerundeten Konturen unter Steifigkeits-Aspekten Vorteile.

Diese Aussparungen 47 lassen sich durch eine Ausdehnung 48 beschreiben, die im wesentlichen parallel zur Bodenplatte 29 bzw. dem Fuß 49 zu bestimmen ist. Hierzu senkrecht weisen die Aussparungen 47 jeweils eine Weite 52 auf. Bevorzugt sind die Aussparungen 47 so gestaltet, dass die Summe der Ausdehnungen 48 mindestens im Bereich von 80 % bis 30 %, vorzugsweise von 70 % bis 40 % und besonders bevorzugt von 60 % bis 65 % der Abmessung 46 liegt.

In Fig. 4 oberhalb der Bodenplatte 29 ist ein Aufsatz 31 in Form eines Steges 35 dargestellt. Der Steg 35 wird mittels Abstandshalter 38 im Randbereich der Bodenplatte 29 nahe dem Behälter 2 fixiert. Allein durch die Vorsehung derartiger Abstandshalter 38 wäre bereits ein Spalt zwischen dem Steg 35 und der Bodenplatte 29 erzeugt, der unter Umständen schon die hier beschriebene, vorteilhafte Beeinflussung der Fluidströmung zur Folge hätte. In Fig. 4 ist nun aber zur Veranschaulichung noch eine besondere Ausführungsform des Steges 35 mit einzelnen Stegen 37 dargestellt. Die Stege 37 bzw. die Abstandshalter 38 haben eine Breite 39, wobei die Summe der Breiten 39 deutlich kleiner als die Länge 36 des Steges 35 ist (beispielsweise kleiner 50%). Bezüglich der oben genannten Prozentangabe sei noch darauf hingewiesen, dass hierbei bevorzugt nur die Abstandshalter 38 bzw. Stege 37 mit ihren Breiten 39 eingehen, die in direktem Kontakt mit der Bodenplatte 29 stehen, also tatsächlich die Strömung über die gesamte Flüssigkeitsschicht behindern. Mit Hilfe der Stege 37 bzw. der Abstandshalter 38 werden demnach Durchlässe 33 gebildet, die bevorzugt ausgehend von der Bodenplatte 29 eine Höhe 34 im Bereich von 1 mm bis 100 mm, vorzugsweise von 5 mm bis 50 mm und besonders bevorzugt von 10 mm bis 30 mm haben.

Fig. 5 zeigt schematische verschiedene Ausführungsformen von Strömungsgleichrichtern 64, die zur einer verbesserten Anströmung des dampfförmigen polymerisationsfähigen Stoffes hin zu einem Trennboden 23 dienen. Grundsätzlich sei zunächst darauf hingewiesen, dass ein solcher Strömungsgleichrichter 64 die Funktion erfüllt, eine gleichmäßige Strömung des polymerisationsfähigen Stoffes hin zu dem mindestens einen Trennboden 23 zu realisieren. Gleichmäßig heißt in diesem Sinne, dass bevorzugt zumindest einer der Faktoren Strömungsgeschwindigkeit und Strömungsrichtung über den Querschnitt des Innenbereichs der Kolonne 1 nahe des Trennbodens 23 nur eine Abweichung im Bereich von weniger als 20 %, insbesondere weniger als 10 % und vorteilhafterweise weniger als 5% beträgt. Das bedeutet beispielsweise, das bei einer vorgebbaren Strömungsgeschwindigkeit des dampfförmigen Stoffes von 2 m/s bis 5 m/s [Meter pro Sekunde] höchstens Abweichungen stromaufwärts des Strömungsgleichrichters von 1 m/s [50% von 2m/s] bis 7,5 m/s [150% von 5 m/s] vorliegen. Betreffen der Strömungsrichtung 25 ist damit gemeint, dass ausgehend von einer senkrecht auf den wenigstens einen Trennboden 23 auftreffende Strömung (senkrechte Anströmrichtung) eine Toleranzweite um diese senkrechte Anströmrichtung von höchstens 180°, bevorzugt 120°, insbesondere 72°, vorteilhafterweise sogar nur 45° und besonders bevorzugt höchstens 20° vorliegt. Diesbezüglich wird insbesondere von einer symmetrischen Anordnung der Toleranzweite bezüglich der senkrechten Anströmrichtung ausgegangen.

Der Strömungsgleichrichter 64 ist vorzugsweise flächig gestaltet und im wesentlichen parallel zu dem mindestens einen Trennboden 23 ausgerichtet bzw. im Innenbereich 5 der Kolonne 1 fixiert. Der Strömungsgleichrichter 64 ist bevorzugt zumindest teilweise aus einem korrosions- und hochtemperaturfesten Material und für ein Fluid durchströmbar. Hierfür sind insbesondere Öffnungen vorgesehen, die einerseits ein Strömungsprofil, bevorzugt im Hinblick auf Geschwindigkeit und/oder Richtung, beeinflusst, andererseits aber ein Verstopfen oder Verschließen der Öffnungen verhindern. Der Strömungsgleichrichter 64 erstreckt sich bevorzugt über den gesamten Innenbereich 5 der Kolonne 1.

Dieser Strömungsgleichrichter 64 umfasst demnach zumindest eines der folgenden Elemente: wenigstens eine Gitterstruktur 67, wenigstens eine Wabenstruktur 68, wenigstens eine Lochplatte 69 oder eine so genannte Packung. Dabei können diese Elemente direkt oder indirekt mit dem Trennboden 23 verbunden sein, insbesondere ein Teil des Trennbodens 23. Die Gitterstruktur 67 umfasst mehrere längliche, faserartige Strukturen, die chaotisch oder wie ein Gewebe miteinander verbunden sind. Als solche länglichen, faserartigen Strukturen eigenen sich beispielsweise beschichtete Metalldrähte. Die Wabenstruktur 68 kann einteilig oder aus einer Mehrzahl von Komponenten hergestellt werden. Die hier gezeigte Ausführungsform umfasst mehrere glatte und strukturierte Blechlagen, die zu einer Wabenstruktur 68 verbunden sind. Die Lochplatte 69 kann neben der dargestellten runden Ausführungsform auch rechteckig, oval, vieleckig oder in einer anderen Weise gestaltet sein. Der Anteil der Löcher beträgt bevorzugt mehr als 30% der Gesamtfläche der Lochplatte 69.
Fig. 6 zeigt ein Detail einer Ausführungsform eines Trennbodens 23 umfassend eine Bodenplatte 29 mit einer Beschichtung 22, die eine gegenüber Stahl verringerte Hafteigenschaft für Flüssigkeiten aufweist. Aus der Darstellung lässt sich erkennen, dass die Beschichtung 22 sich anhand der Kennwerte Schichtdicke 71, Oberflächenrauhigkeit 72 und Porosität 73 beschreiben lässt. Die Beschichtung 22, die bevorzugt Polytetrafluorethylen umfasst, ist auf Kotaktflächen 70 angebracht, die sonst dem direkten Kontakt mit dem polymerisationsfähigen Stoff stehen würden. Dadurch wird verhindert, dass sich der Stoff anlagert und polymerisiert.

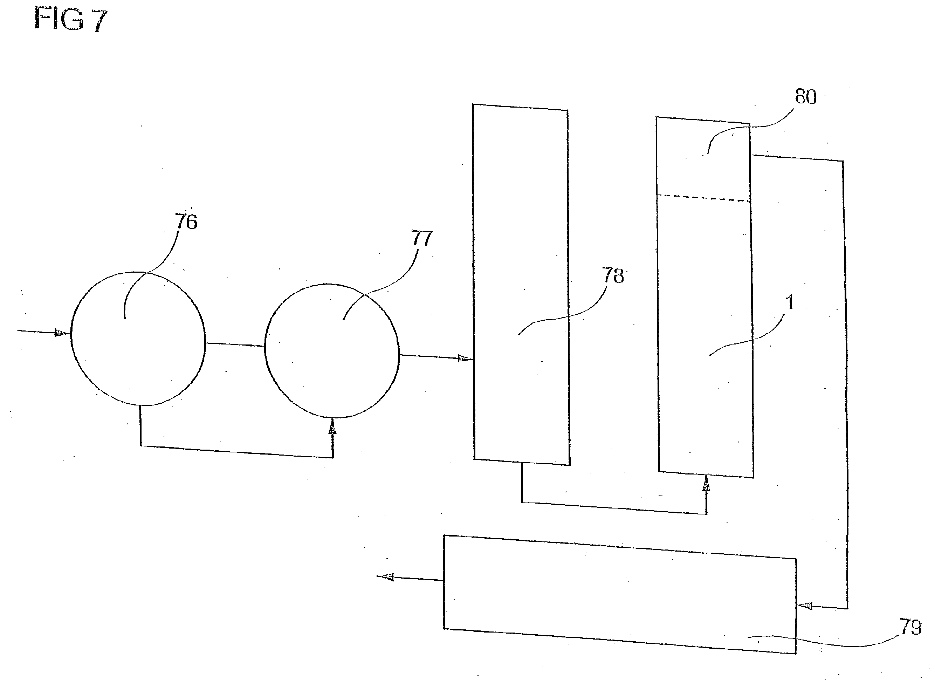

Fig. 7 zeigt schematische eine Anlage zur Herstellung von Acrylsäure die einen ersten Gasphasenoxidationsreaktor 76 zur Oxidation von Propylen zu Acrolein aufweist, der mit einem weiteren Gasphasenoxidationsreaktor 77 verbunden ist, in dem das Acrolein einer weiteren Oxidation zu Acrylsäure unterzogen wird. Das so in dem weiteren Reaktor 77 erhaltenen Acrylsäuregasgemisch wird einem Quencheinrichtung 78 eingespeist, an die sich eine Kolonne 1 mittelbar oder unmittelbar anschließt. An die Kolonne können sich eine oder mehrere weitere Reinigungseinheiten 79 anschließen. Hierzu gehören vorzugsweise Kristallisationsvorrichtungen wie Schichtkristaller, Suspensionskristaller, die mit Waschkolonnen verbunden sind, oder Extraktoren oder Azeotropdestillatoren. Die Reinigungseinheit 79 ist bevorzugt an dem Teil der Kolonne 1 angeordnet, an dem die Acrylsäure in der größten Reinheit anfällt, wobei es sich vorzugsweise um den Kolonnenkopf 80 handelt. Durch diese Gestaltung der Vorrichtung zur Herstellung von Acrylsäure wird diese in einer sehr hohen Reinheit, meist über 99,8 % erhalten. Vergleichbare Vorrichtungsgestaltungen sind ebenfalls für die anderen polymerisationsfähigen Stoffe außer Acrylsäure denkbar.

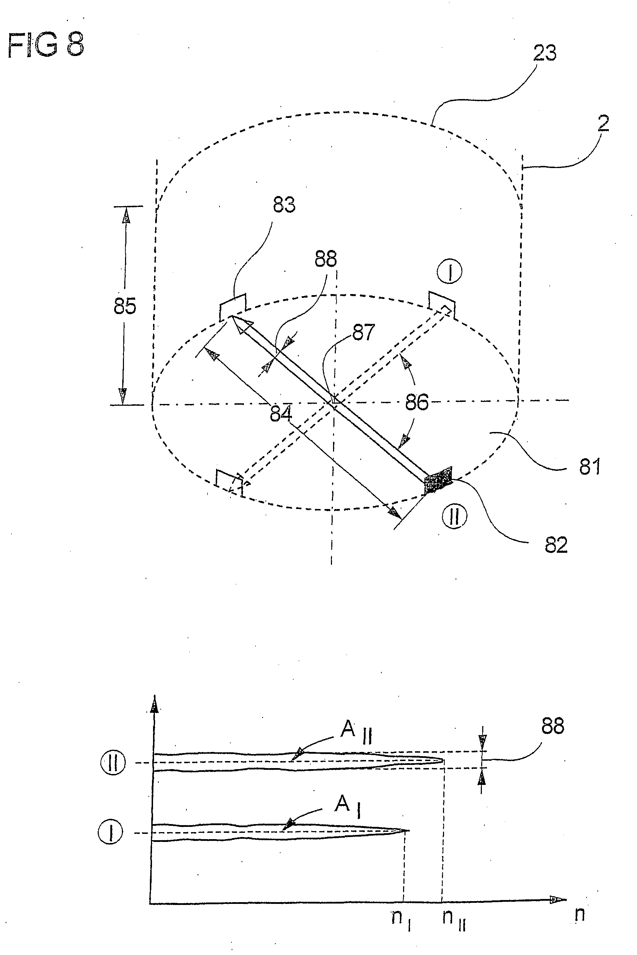

Fig. 8 zeigt schematisch den Aufbau einer Versuchsanordnung zur Bestimmung der Dichteverteilung der Stoff-Gasphase. Gestrichelt dargestellt sind der Behälter 2 sowie der erste Trennboden 23. Mit einer Strecke 85 ist unterhalb des Trennbodens 23 eine imaginäre Ebene 81 dargestellt, in der die Bestimmung der isotropen Dichteverteilung in der Stoff-Gasphase vorgenommen wird. Die Ebene 81 ist bei dem dargestellten Beispiel frei von anderen Bestandteilen des Behälters 2 oder darin angeordneten Komponenten. Auf gegenüberliegenden Bereichen der Ebene 81 sind eine Quelle 82 für eine radioaktive Strahlung sowie ein entsprechender Detektor 83 zur Bestimmung der Menge der auftreffenden radioaktiven Strahlung vorgesehen. Die Quelle 82 sendet einen Strahl durch den Mittelpunkt 87 der Eben 81, die im wesentlichen dem Querschnitt des Behälters 2 entspricht. Weiterhin ist gestrichelt eine weitere Position der Quelle und des Detektors dargestellt und mit (II) gekennzeichnet. Die Positionen (I) und (II) wurden zeitlich nacheinander und mit einer Richtungsänderung 86 versetzt zueinander eingenommen, wobei jeweils ein Mess-Prozess durchgeführt wurde. Dabei hat der Detektor 83 die auftreffende Impuls-Strahlung jeweils gezählt.

Das Mess-Ergebnis ist schematisch mittels zwei balkenähnlicher Graphen unten in Fig. 8 dargestellt. Die Messung erfolgte über einen vorgegebenen Zeitraum und mit einer gewissen Strahl-Breite 88. Der Detektor 83 hat jeweils einen Graphen generiert, der die Verteilung der Zählimpulse (n) über die Strahl-Breite 88 darstellt. Der Maximalwert der ersten Messung (Position I) und der zweiten Messung (Position II) sind in dem Diagramm mit n_{I} und n_{II} gekennzeichnet. Das Integral der Zählimpulse (n) über die Strahl-Breite 88 ist mit A_{I} bzw. A_{II} gekennzeichnet.

Das Wert bzw. die Form des jeweiligen Integrals bzw. der Wert der Zählimpulse ist dabei charakteristisch für die Dichte des durchstrahlten Mediums bzw. der durchstrahlten Stoff-Gasphase. Eine balkenförmige Ausgestaltung der Integrale bzw. ein hoher Wert der Zählimpulse zeigt, dass ein sehr großer Anteil der von der Quelle 82 emittierten Strahlung den Detektor 83 erreicht hat. Umgekehrt deutet ein sehr geringer Wert der Zählimpulse bzw. eine spitze Ausprägung des Graphen auf ein dichteres Medium, welches zumindest teilweise nicht von der radioaktiven Strahlung durchdrungen wird.
Wird eine solche Messung an mehreren Positionen (I, II,...) bei einer zuvor beschriebenen Vorrichtung durchgeführt, wird eine maximale Abweichung der isotropen Dichte innerhalb der Ebene 81 zwischen dem Zulauf 4 und dem ersten Trennbodens 23 höchstens 15% betragen. Bei dem dargestellten Ausführungsbeispiel heißt das, dass der Wert von n_{I} mindestens 70 % von n₂ beträgt. Aufgrund der Tatsache, dass die Anzahl der detektierten Impulse charakteristisch für die Dichte der durchstrahlten Stoff-Gasphase ist, kann dieser Kennwert als Maß für die Dichte herangezogen werden. Damit lässt sich also feststellen, dass eine isotrope Dichtenverteilung gemäß der Erfindung vorliegt.

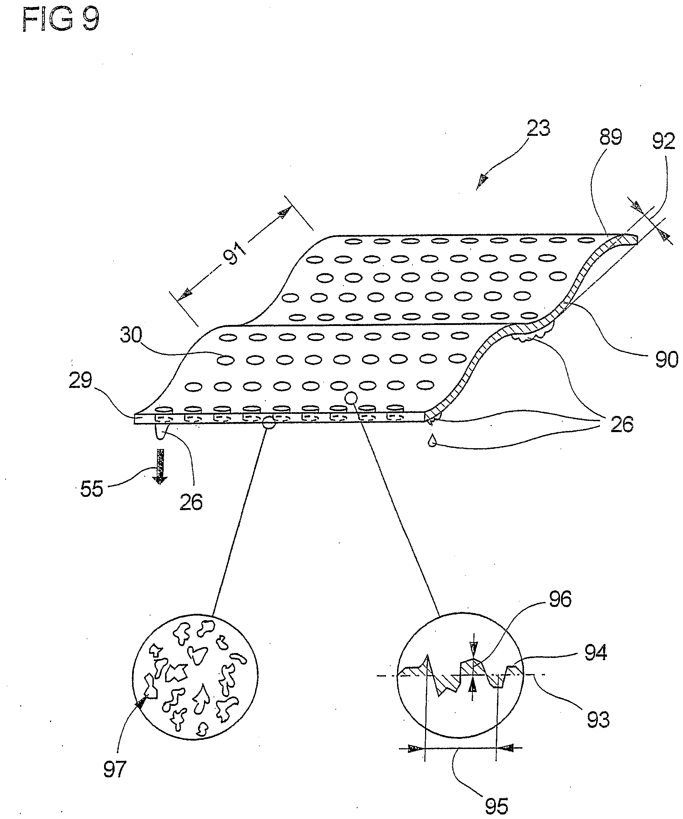

Figur 9 zeigt schematisch ein Detail eine besondere Ausgestaltung einer gewellten Bodenplatte 29 eines Trennbodens 23. Eine solche Bodenplatte 29, insbesondere mit den nachstehenden Eigenschaften, hat eine Vielzahl von Löchern 30 und ist in Kombination mit den hier beschriebenen Ausführungsvarianten aber auch unabhängig davon besonders vorteilhaft. Der Vorteil einer gewellten Bodenplatte 29 ist, dass an der Unterseite der haftende Flüssigkeitstropfen 26 hin zu den Wellentälern 90 ablaufen und sich dort lokal miteinander vermengen. Dies reduziert weiter die Gefahr einer Polymerisation. Gleichzeitig führt diese Ansammlung von Flüssigkeit dazu, dass sich diese letztlich auch ablöst. Bei einer gewellten Ausgestaltung der Bodenplatte 29 kann auf Aufsätze 31, wie sie beispielsweise in den Figuren 2 und 3 gezeigt sind, verzichtet werden, da die Wellenform selbst eine Art Unterteilung bereitstellt, die ein ungewünschtes Hin- und Herströmen der Flüssigkeit verhindert. Bevorzugt werden mehrere oder sogar alle Trennböden 23 einer Kolonne 1 mit solchen gewellten Bodenplatten 29 ausgestattet. Bevorzugt sind dabei benachbart zueinander positionierte Bodenplatten 29 im Hinblick auf die Ausrichtung bzw. Orientierung der Wellenform versetzt zueinander angeordnet, insbesondere in der Form, dass die Wellenberge 89 bzw. Wellentäler 90 der Bodenplatten 29 einen Winkel von ca. 90° bilden.

Besonders bevorzugt ist die Ausgestaltung der Bodenplatte 29 mit einer Wellhöhe 92 im Bereich von 0,5 bis 5,0 cm; insbesondere in einem Bereich von 1,2 bis 1,7 cm. Mit Wellhöhe 92 ist in diesem Zusammenhang der mittlere senkrechte Abstand eines Wellenberges 89 und eines Wellentales 90 zueinander gemeint. Ein Wellenberg 89 liegt dabei in einem horizontalen Abstand von seinem benachbarten Wellental 90 (entspricht der Welllänge 91) von etwa 3,0 cm bis 10 cm, insbesondere liegt die Welllänge 91 in einen Bereich von 4,0 cm bis 6,0 cm.

Eine weitere Verbesserung im Hinblick auf die Reduzierung der Polymerisationsneigung kann durch spezielle Ausgestaltungen von mit dem polymerisationsfähigen Stoff in Kontakt kommenden Flächen der Kolonne 1 erreicht werden. Dies trifft in besonderem Maße für zumindest einem Teil des Trennbodens 23, des Strömungsverteilers 20, des Zulaufes 4, des Strömungsgleichrichters 64 oder des Behälters 2 zu.

Gemäß einer Variante ist zumindest eine dieser vorstehend genannten Flächen bzw. deren Beschichtung wenigstens teilweise mit einem besonders geringen Mittenrauhwert (Rₐ) versehen. Der Mittenrauhwert ist der arithmetische Mittelwert (über einen Bezugsweg 95) der absoluten Beträge der Strecken 96 des Ist-Profils 94 von der Mittellage 93. Hier liegt der Mittenrauwert bevorzugt in einem Bereich kleiner 2,0 µm (Mikrometer), insbesondere in einem Bereich von 0,5 µm bis 1,0 µm. Bei einem solchen Mittenrauwert wird die Haftneigung der Flüssigkeit bezogen auf die von ihr benetzte Oberfläche reduziert, so dass diese schneller abläuft bzw. abtropft. In Fig. 9 ist ein solcher Mittenrauhwert mit Bezug auf die Oberfläche der Bodenplatte 29 beispielhaft und veranschaulichend dargestellt.

Außerdem besteht auch die Möglichkeit (alternativ oder kumulativ), zumindest eine dieser vorstehend genannten Flächen wenigstens teilweise mit einer so genannten selbstreinigenden Oberfläche bzw. Beschichtung zu versehen. Bevorzugt hat diese selbstreinigende Oberfläche eine künstliche, zumindest teilweise hydrophobe Oberflächenstruktur aus Erhebungen und Vertiefungen, wobei die Erhebungen und Vertiefungen durch mittels eines Trägers auf der Oberfläche fixierten Partikel gebildet werden. Vorteilhafterweise zeichnet sich dadurch aus, dass die Partikel eine zerklüftete Struktur mit Erhebungen und/oder Vertiefungen im Nanometerbereich aufweisen (Nanostruktur 97 ist schematisch in Figur 9 dargestellt). Vorzugsweise weisen die Erhöhungen im Mittel eine Höhe von 20 bis 500 nm (Nanometer), besonders bevorzugt von 50 bis 200 nm auf. Der Abstand der Erhöhungen bzw. Vertiefungen auf den Partikeln beträgt vorzugsweise weniger als 500 nm, ganz besonders bevorzugt weniger als 200 nm. Die zerklüfteten Strukturen mit Erhebungen und/oder Vertiefungen im Nanometerbereich können z.B. über Hohlräume, Poren, Riefen, Spitzen und/oder Zacken gebildet werden. Die Partikel selbst weisen eine durchschnittliche Größe von kleiner 50 µm (Mikrometer), vorzugsweise von kleiner 30 µm und ganz besonders bevorzugt von kleiner 20 µm auf. Bevorzugt weisen die Partikel eine BET-Oberfläche von 50 bis 600 m²/g (Quadratmeter pro Gramm) auf. Ganz besonders bevorzugt weisen die Partikel eine BET-Oberfläche von 50 bis 200 m²/g auf. Bei der so genannten "BET-Oberfläche" wird zur Bestimmung der spezifischen Oberfläche auf das weit bekannte Verfahren von BRUNAUER, EMMET und TELLER zurückgegriffen.

Als strukturbildende Partikel können verschiedenste Verbindungen aus vielen Bereichen der Chemie eingesetzt werden. Hierzu sind anorganische Partikel bevorzugt. Vorzugsweise weisen die Partikel zumindest ein Material, ausgewählt aus Silikaten, dotierten Silikaten, Mineralien, Metalloxiden, Kieselsäuren, Polymeren und mit Kieselsäure beschichteten Metallpulvern, auf. Ganz besonders bevorzugt weisen die Partikel pyrogene Kieselsäuren oder Fällungskieselsäuren, insbesondere Aerosile, Al₂O₃, SiO₂, TiO₂, ZrO₂, mit Aerosil R974 ummanteltes Zinkpulver, vorzugsweise mit einer Teilchengröße von 1 µm (Milcrometer) oder pulverförmige Polymere, wie z.B. kryoges, gemahlenes oder sprühgetrocknetes Polytetrafluorethylen (PTFE) oder perfluorierte Copolymere bzw. Copolymere mit Tetrafluorethylen, auf. Derartige Partikel bzw. Beschichtungen zur Generierung selbstreinigender Oberflächen sind beispielsweise über die DEGUSSA AG zu beziehen.

### Messmethoden

Die isotrope Dichte ("richtungsunabhängige" Dichteverteilung) der Stoff-Gasphase bzw. die Abweichung wird zum Beispiel mit einem Verfahren der Firma Ingenieurbüro Bulander & Esper GmbH in Zwingenberg, Deutschland, bestimmt. Dabei wird mittels einer radioaktiven Quelle ein gerichteter Strahl (mit einer vorgegebenen Breite, z.B. 5 cm) hin zu einem Detektor ausgesendet. Dabei befinden sich Quelle und Detektor auf gegenüberliegenden Seiten der Kolonne, so dass sich der Strahl im wesentlichen horizontal durch die Kolonne hindurch erstreckt. Als Quellen werden beispielsweise Kobalt (Co 60) und Cäsium (Cs 137) mit einer Aktivität von 0,3 bis 3,7 GBq eingesetzt.

Der während des Betriebes der Kolonne emittierte Strahl wird vorteilhafterweise mit einem Szintillationsdetektor in Form von Impulsen pro Zeiteinheit gemessen und an eine Auswerte- bzw. Anzeigeeinrichtung weitergeleitet. Grundsätzlich können auch eine Mehrzahl von Detektoren bzw. Quellen vorgesehen sein, die ggf. über den Umfang der Kolonne verteilt angeordnet sind. Letztere Anordnung hat den Vorteil, dass für Vergleichsmessungen in unterschiedliche Richtungen derselbe Versuchsaufbau beibehalten werden kann und nur andere Quellen bzw. Detektoren in Einsatz gelangen, so dass Messungenauigkeiten infolge unsachgemäßer Anbringung vermieden werden können.

Betreffend den Aufbau der Versuchsanordnung wird ergänzend auf die Erläuterungen zu Fig. 8 Bezug genommen.

Während ein solcher radioaktiver Strahl für einen vorgebbaren Zeitraum durch die Stoff-Gasphase hindurch emittiert wird, erkennt ein Zähler des Detektors die auftreffende Strahlung und zählt die Impulse. Die Anzahl der Impulse pro Zeiteinheit ist ein Maß für die Dichte der zwischen Quelle und Detektor befindlichen Materie. Ein hoher Wert charakterisiert eine geringe Dichte, da ein großer Anteil der emittierten Strahlung den Detektor erreicht hat. Dementsprechend ist ein niedriger Wert der gezählten Impulse charakteristisch für eine größere Dichte.

Eine gleichmäßige Strömung der Stoff-Gasphase lässt sich beispielsweise daran erkennen, das in einem Querschnitt die flüssigen und gasförmigen Bestandteile gleichmäßig verteilt sind. Daran ist zu erkennen, dass die Trennböden lokal verstopft sind (so dass dort nur ein geringer Flüssigkeitsanteil in der Stoff-Gasphase vorliegt und somit eine geringere Dichte) oder beispielsweise Bereiche geringer Gasströmungen vorliegen (wo dann aufgrund des geringeren Gegendruckes ein erhöhter Flüssigkeitsstrom und somit eine erhöhte Dichte festzustellen ist).

Zur Feststellung der Isotropie wird nun vorgeschlagen, zunächst eine Messung in einer ersten Richtung in einer Ebene unterhalb des ersten Trennbodens vorzunehmen und über einen vorgegebenen Zeitraum (t; z.B. 5 min) die detektierte Strahlung zu erfassen (n). Um den Einfluss von Betriebsschwankungen der Kolonne zu reduzieren, kann diese Messung auch mehrmals durchgeführt werden, wobei über jeweils den Zeitraum (t) ein Wert (nᵢ) erfasst wird. Anschließend wird ein Mittelwert (N) gebildet und als Referenz für die Isotropie herangezogen. Hierzu sei ergänzend erläutert, das der radioaktive Strahl mit einer bestimmten Breite (z.B. 5 cm) emittiert wird, und der Detektor ggf. eine Auflösung hat, die eine Differenzierung der Messwerte über diese Breite ermöglicht. Dann kann wiederum der Mittelwert oder die Fläche unter dem Graphen (das Integral) als Referenz herangezogen werden, welcher die Impulsrate über die Breite darstellt.

Nachdem nun ein charakteristischer Wert bzw. ein charakteristisches Integral für die detektierte Strahlung erfasst ist, wird nun die vorstehend beschriebene Prozedur auf gleicher Ebene aber in einer davon abweichenden Richtung wiederholt. Die beiden Richtungen schließen dabei eine Winkel ein, der bevorzugt größer als 30° ist, insbesondere sogar größer 40°. Somit sind zumindest 2 solcher Messungen aus verschiedenen Richtungen vorzunehmen, insbesondere sogar wenigstens 3.

Grundsätzlich sollte dabei eine Richtung entlang des Durchmessers der Kolonne gewählt werden, um sicherzustellen, dass die freie Strahllänge durch die Stoff-Gasphase gleich lang ist und sich somit die Werte für die detektierte Strahlung miteinander vergleichen lassen. Dies ist dann deshalb möglich, weil die Strahlung das gleiche Volumen der Stoff-Gasphase durchschritten hat. Natürlich ist auch möglich, eine hiervon abweichende Strahlstrecke zu wählen, es sollte nur sichergestellt sein, dass diese bei jeder Messung die gleiche Länge hat.

Die Ebene kann grundsätzlich beliebig in der Kolonne angeordnet sein und ist bevorzugt im wesentlichen parallel zu mindestens einem Trennboden auszurichten. Für die Überprüfung der Gleichmäßigkeit der Strömung können solche Richtstrahlungen durch einen Trennboden, das Destillat oder durch die Stoff-Gasphase hindurch vorgenommen werden. Zur Charakterisierung des Anströmverhaltens des ersten Trennbodens ist die Ebene bevorzugt in einem Bereich kleiner 200 mm unterhalb des ersten Trennbodens zu wählen. Insbesondere liegt die Ebene in einem Bereich von 100 mm bis 10 mm unterhalb des ersten Trennbodens.

Eine Isotropie der Dichte liegt im Sinne der Erfindung insbesondere dann vor, wenn die Abweichung der erfassten Messwerte (n bzw. N) maximal 15% beträgt. Zur Bestimmung der Abweichung wird ein arithmetischen Mittelwert (M) der Messwerte bestimmt. Er ist bei einer gegebenen Richtungsmessungsanzahl (X) definiert als Quotient aus der Summe der Messwerte je Richtung (n_{X} bzw. N_{X}) und der Anzahl der Messwerte (X). Mit einer maximalen Abweichung von beispielsweise 5 % ist gemeint, dass der höchste Messwert der Impulsrate und der niedrigste Messwert in einem Bereich von 0,95 M bis 1,05 M liegen. Dabei ist bei der hier angegebenen Abweichung bevorzugt schon die Messabweichung infolge kosmischer Umgebungsstrahlung (etwa +/- 50 Zählimpulse bei üblicherweise 3 Sekunden Messdauer und einem Messband von ca. 50 mm) berücksichtigt.

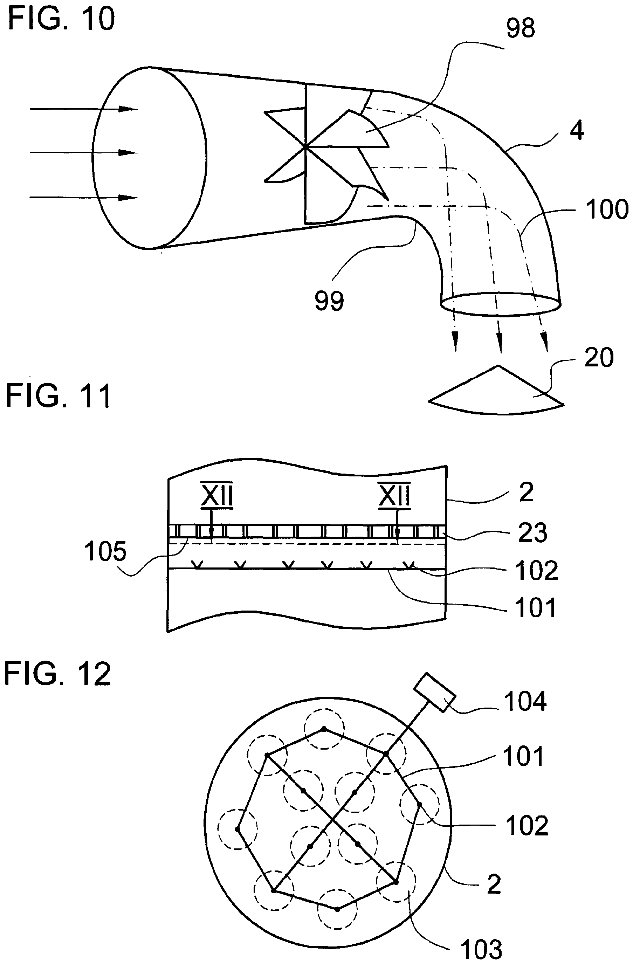

Fig. 10 zeigt schematisch und perspektivisch eine weitere Ausführungsform des Zulaufs 4 mit einem Strömungsmischer 98 als besondere Ausgestaltung eines Strömungsbeeinflussers. Der dargestellte Zulauf 4 weist eine Krümmung 99 auf, in der der polymerisationsfähige Stoff umgelenkt wird. Würde der polymerisationsfähige Stoff ohne einen Strömungsbeeinflusser frei durch einen solchen Zulauf 4 strömen, käme es aufgrund der Krümmung 99 zu einer ungleichmäßigen Geschwindigkeitsverteilung der Strömung über den Querschnitt des Zulaufs 4. Grund hierfür sind Strömungswirbel und Rückströmungen im Bereich der Krümmung 99. Um dies zu vermeiden ist es auch möglich, einen Strömungsmischer 98 stromaufwärts in der Nähe (bevorzugt unmittelbar vor) der Krümmung 99 vorzusehen. Ein solcher Strömungsmischer 98 unterteilt den anströmenden polymerisationsfähigen Stoff in mehrere Strömungsfäden 100 und lenkt diese so ab, dass sie im wesentlichen die gleiche Wegstrecke durch die Krümmung 99 hindurch zurücklegen. Dabei wird der polymerisationsfähige Stoff bevorzugt zumindest teilweise in Rotation versetzt. Damit kann eine einheitliche Strömung ohne Pulsationen und Rückvermischungen generiert werden, so dass der Querschnitt des Zulaufs 4 auch nach der Krümmung 99 gleichmäßig bestömt wird und der polymerisationsfähige Stoff gleichmäßig verteilt beispielsweise auf den Strömungsverteiler 20 auftrifft. Es sei schließlich noch darauf hingewiesen, dass die Bereitstellung eines solchen Strömungsbeeinflussers bzw. Strömungsmischers 98 an mehreren Krümmungen 99 des Zulaufs 4 erfolgen kann.

Fig. 11 veranschaulicht einen Teilausschnitt eines Behälters 2 mit einer Sprühanlage 101 im Querschnitt, wobei in Fig. 12 eine Draufsicht auf die in Fig. 11 gezeigte Sprühanlage 101 gezeigt ist. Der Behälter 2 ist mit einem Trennboden 23 ausgeführt, dessen Unterseite 105 (insbesondere während des Betriebes der Kolonne 1) mit einer Sprühanlage 101 gereinigt wird. Eine solche Sprühanlage 101 ist bevorzugt zumindest für den untersten Trennboden 23 des Behälters 2 vorgesehen, wenn dieser eine Mehrzahl von übereinander angeordneten Trennböden 23 aufweist. Durch diesen Trennboden 23 strömt eine Flüssigkeit des pölymerisationsfähigen Stoffes mit einer bestimmten Zusammensetzung, die anschließend beispielsweise in einem Sammelbecken 24 des Behälters 2 aufgefangen wird. Vorteilhafterweise wird nun vorgeschlagen, diese Flüssigkeit über eine Liefervorrichtung 104 der Sprühanlage 101 zur Verfügung zu stellen und damit die Unterseite des Trennbodens 23 zu reinigen. Die Verwendung dieser Flüssigkeit hat den Vorteil, dass keine wesentliche Beeinflussung der Destillation im untersten Trennboden 23 stattfindet. Mit dieser Sprühanlage 101 werden an der Unterseite 105 des Trennbodens 23 haftende (ggf. schon teilweise polymerisierte) Bestandteile des polymerisationsfähigen Stoffes wirksam entfernt.

Die Sprühanlage 101 selbst kann mit einer Mehrzahl von Düsen 102 ausgeführt sein. Diese sind so ausgeführt, dass eine im wesentlichen gleichmäßige Reinigung des Trennbodens 23 über dessen gesamten Querschnitt erfolgen kann. Dabei kann die Anordnung und/oder die Art der Düsen entsprechend ausgewählt sein. Die Fig. 12 zeigt schematisch eine mögliche Ausführungsform der Sprühanlage 101 mit gleichmäßig verteilten Düsen 102, die einen im wesentlichen gleichartigen Sprühbereich 103 aufweisen. Eine solche Ausgestaltung der Sprühanlage 101 ist technisch und wirtschaftliche einfach, allerdings nicht zwingend erforderlich. Die Düsen 102 sind hier so angeordnet, dass sich die Sprühbereiche 103 im wesentlichen nicht überlagern, aber auch dies ist nicht zwingend. Als Düsen 102 kommen sowohl einfache Öffnungen in der Sprühanlage 101 als auch separate Düsenbauteile (bevorzugt) in Betracht.

### Bezugszeichenliste

- 1: Kolonne
- 2: Behälter
- 3: Anschluss
- 4: Zulauf
- 5: Innenbereich
- 6: Außenfläche
- 7: Teilabschnitt
- 8: Unterboden
- 9: Teilbereich
- 10: Umhüllung
- 11: Schicht
- 12: Innenfläche
- 13: Eintrittsöffnung
- 14: Austrittsöffnung
- 15: Strömungsbeeinflusser
- 16: Leitblech
- 17: Kanal
- 18: Strecke
- 19: Mittelachse
- 20: Strömungsverteiler
- 21: Spitze
- 22: Beschichtung
- 23: Trennboden
- 24: Sammelbecken
- 25: Strömungsrichtung
- 26: Tropfen
- 27: Erhitzer
- 28: Pumpe
- 29: Bodenplatte
- 30: Öffnung
- 31: Aufsatz
- 32: Gruppe
- 33: Durchlass
- 34: Höhe
- 35: Steg
- 36: Länge
- 37: Strebe
- 38: Abstandshalter
- 39: Breite
- 40: Sektor
- 41: Deckplatte
- 42: Entfernung
- 43: Halterung
- 44: Träger
- 45: Seite
- 46: Abmessung
- 47: Aussparung
- 48: Ausdehnung
- 49: Fuß
- 50: Dimension
- 51: Abstand
- 52: Weite
- 53: Vorsprung
- 54: Pfeil
- 55: Gravitation
- 56: Flüssigkeitsschicht
- 57: Dampfblasenschicht
- 58: Tropfenschicht
- 59: Blase
- 60: Flüssigkeit
- 61: Leitfläche
- 62: Zentrumsachse
- 63: Abrisskante
- 64: Strömungsgleichrichter
- 65: Verbindungselement
- 66: Mantelfläche
- 67: Gitterstruktur
- 68: Wabenstruktur
- 69: Lochplatte
- 70: Kontaktfläche
- 71: Schichtdicke
- 72: Oberflächenrauhigkeit
- 73: Porosität
- 74: Distanz
- 75: Flüssigkeitsspiegel
- 76: ersten Gasphasenoxidationsreaktor
- 77: weiteren Gasphasenoxidationsreaktor
- 78: Quencheinrichtung
- 79: Reinigungseinheiten
- 80: Kolonnenkopf
- 81: Ebene
- 82: Quelle
- 83: Detektor
- 84: Weg
- 85: Strecke
- 86: Richtungsänderung
- 87: Mittelpunkt
- 88: Strahl-Breite
- 89: Wellenberg
- 90: Wellental
- 91: Welllänge
- 92: Wellhöhe
- 93: Mittellage
- 94: Ist-Profil
- 95: Bezugsweg
- 96: Strecke
- 97: Nanostruktur
- 98: Strömungsmischer
- 99: Krümmung
- 100: Strömungsfaden
- 101: Sprühanlage
- 102: Düse
- 103: Sprühbereich
- 104: Liefervorrichtung

## Patentansprüche

1. Verfahren zur Aufreinigung eines polymerisationsfähigen Stoffes, wobei der polymerisationsfähige Stoff in einer Kolonne (1) mit mindestens einem Trennboden (23) durch einen Zulauf (4) als eine Stoff-Flüssigkeit eingebracht und in einem Innenbereich (5) in eine Stoff-Gasphase überführt wird, wobei die Stoff-Gasphase einen oberhalb des Zulaufs (4) angeordneten ersten Trennboden (23) mit einer isotropen Dichte anströmt, wobei innerhalb einer Ebene zwischen dem Zulauf (4) und dem ersten Trennboden (23) die maximale Abweichung um einen Mittelwert der isotropen Dichte höchstens 15% beträgt,
wobei der Trennboden (23) zumindest eine Bodenplatte (29) mit einer Vielzahl von Öffnungen (30) und mindestens einen Aufsatz (31) umfasst, **dadurch gekennzeichnet, dass** der Aufsatz (31) die Vielzahl von Öffnungen (30) in Gruppen (32) aufteilt, wobei mit dem mindestens einen Aufsatz (31) für ein Fluid durchströmbare Durchlässe (33) gebildet sind, und wobei der polymerisationsfähige Stoff (Meth)Acrylsäure ist.

2. Verfahren nach Anspruch 1, wobei im Innenbereich ein Unterdruck herrscht.

3. Verfahren nach einem der Ansprüche 1 bis 2, wobei die Stoff-Flüssigkeit überhitzt ist.

4. Verfahren nach Anspruch 1, **dadurch gekennzeichnet, dass** die Durchlässe (33) sich von der Bodenplatte (29) aus über eine Höhe (34) von 1 mm bis 100 mm erstrecken.

5. Verfahren nach Anspruch 1 oder 4, **dadurch gekennzeichnet, dass** der mindestens eine Aufsatz (31) zumindest einen gerade verlaufenden Steg (35) umfasst.

6. Verfahren nach Anspruch 5, **dadurch gekennzeichnet, dass** dieser zumindest eine der folgenden Konfigurationen aufweist:
(a) es sind mehrere Aufsätze (31) mit einem Steg (35) vorgesehen.
(b) es ist ein Aufsatz (31) mit mehreren, vorzugsweise miteinander verbundenen, Stegen (35) vorgesehen.

7. Verfahren nach Anspruch 6, **dadurch gekennzeichnet, dass** der mindestens eine Steg (35) eine Länge (36) hat und die Mehrzahl von Durchlässen (30) zumindest teilweise durch wenigstens eines der folgenden Elemente begrenzt ist:
(a) wenigstens eine Strebe (37), die Teil des Steges (35) ist,
(b) wenigstens einen Abstandshalter (38), der ein separates Teil ist,
wobei diese Elemente eine Breite (39) aufweisen, und die Summe der Breiten (39) der Elemente kleiner als 80 % der Länge (26) des Steges (35) ist.

8. Verfahren nach einem der vorhergehenden Ansprüche 4 bis 7, **dadurch gekennzeichnet, dass** der mindestens eine Aufsatz (31) Sektoren (40) der Bodenplatte (29) mit jeweils einer Gruppe (32) von Öffnungen (30) bildet, wobei die Sektoren (40) bevorzugt eine Fläche im Bereich von 1,2 m2 bis 0,3 m2 umfassen.

9. Verfahren nach einem der vorhergehenden Ansprüche 4 bis 8, **dadurch gekennzeichnet, dass** dieser eine Deckplatte (41) hat, wobei diese bevorzugt in einer Entfernung (42) von 60 mm bis 200 mm von der Bodenplatte (29) angeordnet ist und insbesondere ebenfalls eine Vielzahl von Öffnungen (30) aufweist.

10. Verfahren nach einem der vorhergehenden Ansprüche 4 bis 9, **dadurch gekennzeichnet, dass** dieser eine Halterung (43) umfasst, wobei die Halterung (43) wenigstens einen massiven Träger (44) aufweist, welcher mit der dem mindestens einen Aufsatz (31) abgewandten Seite (45) der Bodenplatte (29) in Kontakt ist, bevorzugt über eine Abmessung (46) im Bereich von 200 mm bis 1000 mm.

11. Verfahren nach Anspruch 10, **dadurch gekennzeichnet, dass** der wenigstens eine Träger (44) nahe der Bodenplatte (29) mindestens eine Aussparung (47) mit einer Ausdehnung (48) hat, wobei die Summe der Ausdehnungen (48) mindestens im Bereich von 80 % bis 30 % der Abmessung (46) liegt.

12. Verfahren nach Anspruch 11, **dadurch gekennzeichnet, dass** der wenigstens eine Träger (44) T-förmig gestaltet ist, wobei die mindestens eine Aussparung (47) in dem im wesentlichen senkrecht zur Bodenplatte (29) liegenden Trägerteil angeordnet ist.

13. Verfahren nach Anspruch 12, **dadurch gekennzeichnet, dass** der wenigstens eine T-förmige Träger (44) einen parallel zur Bodenplatte (29) angeordneten Fuß (49) mit einer Dimension (50) von 50 mm bis 100 mm hat, wobei die mindestens eine Aussparung (47) in einem Abstand (51) kleiner 3 mm, insbesondere direkt am Fuß (49) anschließend, angeordnet ist und bevorzugt eine Weite (52) von 40 mm bis 120 mm hat.

14. Verfahren nach einem der Ansprüche 10 bis 13, **dadurch gekennzeichnet, dass** der mindestens eine Träger (44) und der mindestens eine Aufsatz (31) senkrecht zueinander angeordnet sind.

15. Verfahren nach einem der vorhergehenden Ansprüche 4 bis 14, **dadurch gekennzeichnet, dass** dieser zumindest teilweise mit einer Beschichtung (22) versehen ist, die eine gegenüber Stahl verbesserte Gleiteigenschaft für Flüssigkeiten aufweist.

## Claims

1. Process for purifying a polymerizable substance, wherein the polymerizable substance, in a column (1) having at least one separation tray (23), is introduced through a feed (4) as a substance liquid and converted to a substance gas phase in an interior (5), wherein the substance gas phase flows with an isotropic density toward a first separation tray (23) disposed above the feed (4), wherein the maximum variation about an average isotropic density value within a layer between the feed (4) and the first separation tray (23) is at most 15%,
wherein the separation tray (23) comprises at least one base plate (29) having a multitude of openings (30) and at least one top part (31), **characterized in that** the top part (31) divides the multitude of openings (30) into groups (32), wherein passages (33) through which a fluid can flow are formed by the at least one top part (31), and wherein the polymerizable substance is (meth)acrylic acid.

2. Process according to Claim 1, wherein there is a reduced pressure in the interior.

3. Process according to either of Claims 1 and 2, wherein the substance liquid has been superheated.

4. Process according to Claim 1, **characterized in that** the passages (33) extend from the base plate (29) over a height (34) of 1 mm to 100 mm.

5. Process according to Claim 1 or 4, **characterized in that** the at least one top part (31) comprises at least one fillet (35) that runs in a straight line.

6. Process according to Claim 5, **characterized in that** said top part has at least one of the following configurations:
(a) multiple top parts (31) with a fillet (35) are provided,
(b) one top part (31) with multiple, preferably interconnected fillets (35) is provided.

7. Process according to Claim 6, **characterized in that** the at least one top part (35) has a length (36) and the multitude of passages (30) is bounded at least partly by at least one of the following elements:
(a) at least one strut (37) that is part of the fillet (35),
(b) at least one spacer (38) which is a separate part,
wherein these elements have a width (39), and the sum total of the widths (39) of the elements is less than 80% of the length (26) of the fillet (35).

8. Process according to any of the preceding Claims 4 to 7, **characterized in that** the at least one top part (31) forms sectors (40) of the base plate (29) each with a group (32) of openings (30), where the sectors (40) preferably encompass an area in the range from 1.2 m² to 0.3 m².

9. Process according to any of the preceding Claims 4 to 8, **characterized in that** said top part has a top plate (41) which is preferably disposed at a distance (42) of 60 mm to 200 mm from the baseplate (29) and especially likewise has a multitude of openings (30).

10. Process according to any of the preceding Claims 4 to 9, **characterized in that** said top part comprises a holder (43), where the holder (43) has at least one solid carrier (44) which is in contact with the side (45) of the baseplate (29) remote from the at least one top part (31), preferably over a dimension (46) in the range from 200 mm to 1000 mm.

11. Process according to Claim 10, **characterized in that** the at least one carrier (44), close to the baseplate (29), has at least one cutout (47) with an extent (48), where the sum total of the extents (48) is at least in the range from 80% to 30% of the dimension (46) .

12. Process according to Claim 11, **characterized in that** the at least one carrier (44) is in T-shaped configuration, where the at least one cutout (47) is disposed in the part of the carrier essentially at right angles to the baseplate (29).

13. Process according to Claim 12, **characterized in that** the at least one T-shaped carrier (44) has a foot (49) which is arranged parallel to the base plate (29) and has a dimension (50) of 50 mm to 100 mm, where the at least one cutout (47) is disposed at a distance (51) of less than 3 mm, especially directly adjoining the foot (49), and preferably has a width (52) of 40 mm to 120 mm.

14. Process according to any of Claims 10 to 13, **characterized in that** the at least one carrier (44) and the at least one top part (31) are arranged at right angles to one another.

15. Process according to any of the preceding Claims 4 to 14, **characterized in that** said top part has been provided at least partly with a coating (22) having an improved slip property for liquids compared to steel.

## Revendications

1. Procédé de purification d'une matière polymérisable, selon lequel la matière polymérisable est introduite dans une colonne (1) comprenant au moins un plateau de séparation (23) par une alimentation (4) sous la forme d'une matière liquide et est transformée en une matière en phase gazeuse dans une zone intérieure (5), la matière en phase gazeuse arrivant sur un premier plateau de séparation (23) agencé au-dessus de l'alimentation (4) avec une densité isotrope, la déviation maximale d'une valeur moyenne de la densité isotrope dans un plan entre l'alimentation (4) et le premier plateau de séparation (23) étant d'au plus 15 %, le plateau de séparation (23) comprenant au moins une plaque de plateau (29) qui comprend une pluralité d'ouvertures (30) et au moins une partie supérieure (31), **caractérisé en ce que** la partie supérieure (31) divise la pluralité d'ouvertures (30) en groupes (32), des passages (33) pouvant être traversés par un fluide étant formés avec ladite au moins une partie supérieure (31), et la matière polymérisable étant l'acide (méth)acrylique.

2. Procédé selon la revendication 1, dans lequel une sous-pression règne dans la zone intérieure.

3. Procédé selon l'une quelconque des revendications 1 à 2, dans lequel la matière liquide est surchauffée.

4. Procédé selon la revendication 1, **caractérisé en ce que** les passages (33) s'étendent depuis la plaque de plateau (29) sur une hauteur (34) de 1 mm à 100 mm.

5. Procédé selon la revendication 1 ou 4, **caractérisé en ce que** ladite au moins une partie supérieure (31) comprend au moins une traverse droite (35).

6. Procédé selon la revendication 5, **caractérisé en ce que** celui-ci présente au moins une des configurations suivantes :
(a) plusieurs parties supérieures (31) munies d'une traverse (35) sont prévues,
(b) une partie supérieure (31) munie de plusieurs traverses (35), de préférence reliées les unes avec les autres, est prévue.

7. Procédé selon la revendication 6, **caractérisé en ce que** ladite au moins une traverse (35) a une longueur (36) et la pluralité de passages (30) est au moins partiellement délimitée par au moins un des éléments suivants :
(a) au moins une entretoise (37), qui fait partie de la traverse (35),
(b) au moins un espaceur (38), qui est une partie séparée,
ces éléments présentant une largeur (39), et la somme des largeurs (39) des éléments étant inférieure à 80 % de la longueur (26) de la traverse (35).

8. Procédé selon l'une quelconque des revendications 4 à 7 précédentes, **caractérisé en ce que** ladite au moins une partie supérieure (31) forme des secteurs (40) de la plaque de plateau (29) comprenant à chaque fois un groupe (32) d'ouvertures (30), les secteurs (40) comprenant de préférence une surface dans la plage allant de 1,2 m² à 0,3 m².

9. Procédé selon l'une quelconque des revendications 4 à 8 précédentes, **caractérisé en ce que** celui-ci comprend une plaque de recouvrement (41), celle-ci étant de préférence agencée à un écart (42) de 60 mm à 200 mm de la plaque de plateau (29), et comprenant notamment également une pluralité d'ouvertures (30).

10. Procédé selon l'une quelconque des revendications 4 à 9 précédentes, **caractérisé en ce que** celui-ci comprend une fixation (43), la fixation (43) comprenant au moins un support massif (44), qui est en contact avec le côté (45) de la plaque de plateau (29) opposé à ladite au moins une partie supérieure (31), de préférence sur une dimension (46) dans la plage allant de 200 mm à 1 000 mm.

11. Procédé selon la revendication 10, **caractérisé en ce que** ledit au moins un support (44) comprend à proximité de la plaque de plateau (29) au moins un évidement (47) présentant une étendue (48), la somme des étendues (48) se situant au moins dans la plage allant de 80 % à 30 % de la dimension (46).

12. Procédé selon la revendication 11, **caractérisé en ce que** ledit au moins un support (44) est configuré en forme de T, ledit au moins un évidement (47) étant agencé dans la partie du support qui se situe essentiellement perpendiculairement à la plaque de plateau (29).

13. Procédé selon la revendication 12, **caractérisé en ce que** ledit au moins un support en forme de T (44) comprend une base (49) agencée parallèlement à la plaque de plateau (29), d'une dimension (50) de 50 mm à 100 mm, ledit au moins un évidement (47) étant agencé à une distance (51) inférieure à 3 mm, notamment directement adjacente à la base (49), et présentant de préférence une largeur (52) de 40 mm à 120 mm.

14. Procédé selon l'une quelconque des revendications 10 à 13, **caractérisé en ce que** ledit au moins un support (44) et ladite au moins une partie supérieure (31) sont agencés parallèlement l'un à l'autre.

15. Procédé selon l'une quelconque des revendications 4 à 14 précédentes, **caractérisé en ce que** celui-ci est au moins partiellement muni d'un revêtement (22), qui présente des propriétés de glissement pour les liquides améliorées en comparaison de l'acier.
